# EUROPÄISCHE PATENTSCHRIFT

(11) **EP 0 819 330 B1**
(45) Veröffentlichungstag und Bekanntmachung des Hinweises auf die Patenterteilung: **06.06.2001**
(21) Anmeldenummer: 96907235.4
(22) Anmeldetag: 02.04.1996
(51) Int. Cl.: H02K 7/09, F04D 29/04, F04D 13/06, F04D 5/00, F04D 29/42, F04D 1/00, A61M 1/10

(54) **ROTATIONSMASCHINE MIT ELEKTROMAGNETISCHEM DREHANTRIEB**
ROTARY MACHINE WITH AN ELECTROMAGNETIC ROTARY DRIVE
MACHINE ROTATIVE AVEC SYSTEME ELECTROMAGNETIQUE D'ENTRAINEMENT EN ROTATION

(30) Priorität: 03.04.1995 CH 94395
(43) Veröffentlichungstag der Anmeldung: 21.01.1998
(60) Teilanmeldung: 00112231.6
(73) Patentinhaber: Levitronix LLC, Waltham, MA 02451 (US)
(72) Erfinder: SCHÖB, Reto, CH-8604 Volketswil (CH); HUGEL, Jörg, CH-8008 Zürich (CH); MENDLER, Niklaus, D-82335 Berg (DE)
(74) Vertreter: Sulzer Management AG
(86) Internationale Anmeldenummer: CH9600117
(87) Internationale Veröffentlichungsnummer: WO9631934

(56) Entgegenhaltungen:
- EP-A- 0 378 251
- WO-A-88/07842
- DE-A- 2 406 790
- DE-A- 2 457 084
- DE-U- 9 108 432
- FR-A- 2 681 384
- JP-A- 3 107 615
- US-A- 4 944 748
- US-A- 5 237 229

## Beschreibung

Die Erfindung betrifft eine Rotationsmaschine nach dem Oberbegriff des Patentanspruchs 1. Weiterhin bezieht sich die Erfindung auch auf derartige als Pumpen und Rührwerke ausgeführte Rotationsmaschinen.

Kreiselpumpen mit hermetisch abgeschlossenen Pumpengehäusen kommen dann zur Verwendung, wenn eine vollständige Trennung zwischen dem zu fördernden Fluid und der Umgebung gefordert wird. Obwohl diese Forderung mit Schlauchquetschpumpen leichter zu erfüllen wäre als mit Rotationspumpen, sind Schlauchquetschpumpen, sog. peristaltische Pumpen, häufig nicht einsetzbar, weil durch ihre spezifische Konstruktion bei ihrem Betrieb Scherkräfte auf das Fluid wirken, durch welche es in seiner Struktur beeinträchtigt wird. Insbesondere in pharmazeutischen und medizinischen Anwendungsbereichen, wo mechanisch empfindliche Fluide mit langen Molekülketten oder mit Zellen, die empfindliche Zellwände besitzen, zu fördern sind, besteht die Notwendigkeit, Rotationspumpen einzusetzen. Beispielsweise besteht bei der Förderung von Blut die Gefahr, dass infolge solcher Scherkräfte eine Hämolyse auftritt, was dazu führt, dass das Blut unbrauchbar wird. Anders als in Kolbenpumpen sind die Fluide in Kreiselpumpen kaum Scherkräften ausgesetzt, so dass lange Moleküle und empfindliche Zellen bei ihrer Förderung geschont werden.

Eine strikte stoffliche Isolierung des zu fördernden Fluids kann aus zwei verschiedenen Gründen notwendig sein: einerseits soll dadurch ein Ausströmen in die Umgebung selbst geringster Fluidmengen verunmöglicht werden, wenn kontaminierende Stoffe gefördert werden; anderseits soll ein Eindringen von Fremdstoffen irgendwelcher Art in das Fluid verhindert werden, wenn dieses höchsten Anforderungen bezüglich seiner Reinheit genügen muss, was vor allem bei einem Einsatz der Pumpe im chemischen, pharmazeutischen oder medizinischen Bereich der Fall ist. Besonders für diese Anwendungsbereiche besteht die stoffliche Isolierung des zu fördernden Fluides nicht nur darin, den Zutritt von Umgebungsluft zu verunmöglichen; es soll auch verhindert werden, dass Abriebpartikel von relativ zueinander bewegten Bauteilen der Antriebsvorrichtung, der Lagervorrichtung oder einer Dichtungsanordnung oder Schmierstoffe in das Fluid gelangen.

Während Operationen am offenen Herzen werden zur Aufrechterhaltung des Blutkreislaufes Pumpen der eingangs genannten Art verwendet, wobei das zu fördernde Fluid das Blut des Patienten ist. Es versteht sich von selbst, dass dabei höchste Ansprüche bezüglich der Reinhaltung des zu fördernden Fluids gestellt werden.

Bei der Verwendung von konventionellen Antriebsvorrichtungen, Lagervorrichtungen und Gleitringdichtungen war es nicht möglich, das Pumpengehäuse vollständig gegen die Umgebung abzudichten und gleichzeitig die Entstehung von Abriebpartikeln zwischen relativ zueinander bewegten Bauteilen und den Zutritt von solchen Abriebpartikel und von Schmierstoffen zum Fluid zu verhindern.

Mit den seit längerer Zeit bekannten magnetischen Lagern wurde es möglich, konventionelle Wälz- oder Gleitlager durch eine Lagervorrichtung zu ersetzen, der nicht nur eine berührungsfreie Lagerung, beispielsweise in der Art eines Auftriebs-Gleitlagers, sondern auch eine ungeschmierte Lagerung ermöglicht.

Einen weiteren Fortschritt in derselben Richtung ist die Entwicklung von Drehstrommotoren mit einer Trennung zwischen dem Stator und dem Rotor, sog. Spaltrohrmotoren.

EP-0 551 435 beispielsweise beschreibt einen elektromagnetischen Drehantrieb für eine Rotationspumpe mit einem hermetisch abgeschlossenen Pumpengehäuse und einem Pumpenrotor, der mittels einer berührungsfreien Lagervorrichtung gelagert und über einen Spaltrohrmotor angetrieben wird.

Dieser elektromagnetische Drehantrieb für die Pumpe ist aufwendig konstruiert und relativ voluminös, wobei insbesondere ihre axiale Abmessung gross ist. Die Magnetlager brauchen viel Platz.

WO-A-88/07842 beschreibt eine Axialblutpumpe mit einem Rotor, der sowohl antreibender Rotor als auch angetriebener Rotor ist. Der Rotor weist längliche Gestalt auf und wird mit Hilfe von zwei Statoren gelagert und angetrieben. Die beiden Statoren erlauben auch eine aktive Stabilisierung des Rotors gegen Verkippen. Die Pumpe ist jedoch relativ voluminös und sowohl vom konstruktiven als auch vom regelungstechnischen Aufwand her vergleichsweise aufwendig.

In der JP-A-3107615 ist eine Rotationsmaschine offenbart, die einen Schaft aufweist, auf welchem zwei Rotoren angeordnet sind, die Bestandteil einer jeweiligen Magnetlagervorrichtung sind. Die Magnetlagervorrichtungen können entweder beide oder aber auch nur eine davon als Lager-/Antriebsvorrichtung ausgebildet sein. In dem Fall, in welchem nur eine Magnetlagervorrichtung als Lager-/Antriebsvorrichtung ausgebildet ist, ist die andere Magnetlagervorrichtung als konventionelles Magnetlager (Radiallager) ausgebildet.

Die Aufgabe der Erfindung ist es, eine verbesserte, kompakte und einfache elektromagnetische Rotationsmaschine, z.B. eine Rotationspumpe, Mischer, Rührwerke und andere Einrichtungen, zu schaffen.

Diese Aufgabe wird erfindungsgemäss mit einer elektromagnetischen Rotationsmaschine gelöst, die die Merkmale von Patentanspruch 1 aufweist. Die abhängigen Ansprüche beziehen sich auf bevorzugte Weiterbildungen der Erfindung.

Bei der erfindungsgemässen elektromagnetischen Rotationsmaschine, z.B. einer Rotationspumpe, u.s.w., bildet die Lagervorrichtung und die Antriebsvorrichtung nach dem Prinzip des sogenannten lagerlosen Motors eine kombinierte, berührungsfreie und schmierstofffreie Lager / Antriebs-Vorrichtung, deren kombinierter Lager/Antriebsrotor so ausgebildet ist, dass er neben der Funktion des Rotors des Elektromotors auch die Funktion des Rotors der Einrichtung, also beispielsweise eines Pumpenrotors, Mischerrotors oder Rührwerksrotors übernimmt. Man nennt solche Rotoren Integralrotoren.

Das Prinzip des 'lagerlosen' Motors besteht darin, dass einerseits der Rotor einer Drehfeldmaschine und eine gelagerte Wellenanordnung den erwähnten kombinierten Lager/Antriebs- Rotor ergeben und dass anderseits der Stator der Drehfeldmaschine und der nicht drehende Teil einer Lagervorrichtung einen Lager/Antriebs-Stator ergeben. Bei der Anordnung eines 'lagerlosen Motors' als Lager/Antriebs-Vorrichtung einer Rotationspumpe sind das Pumpengehäuse und der Lager/Antriebs-Stator im Betrieb ortsfest, während der Lager/Antriebs-Rotor sowie der Pumpenrotor zwei drehbare Bauteile wären, welche beiden Bauteile zusammen den Integralrotor bilden.
Erfindungsgemäss ist nun nur noch ein einziger Stator vorgesehen, wobei dieser einzige Stator den Integralrotor sowohl lagert als auch antreibt, und wobei die axiale Länge des antreibenden Rotors kleiner oder gleich dem halben Durchmesser dieses Rotors ist und des Integralrotor axial sowie gegen Verkippung gegenüber der Statorebene passiv durch Reluktanzkräfte stabilisiert ist.

Vorteilhafterweise besitzt die Rotationsmaschine eine Antriebswicklung mit der Polpaarzahl p und eine Steuerwicklung mit der Polpaarzahl p+1 oder p-1; die Drehung des Integralrotors um seine Rotationsachse wird über die Antriebswicklung aktiv gesteuert oder geregelt, und die Position des Integralrotors in der senkrecht zur Rotationsachse verlaufenden Ebene wird durch die Steuerwicklung aktiv geregelt und stabilisiert. Die Position des Integralrotors in Richtung der Rotationsachse und seine Verkippung gegenüber der genannten Ebene werden durch Reluktanzkräfte passiv stabilisiert.
Mit der erfindungsgemässen Konstruktion, welche durch die Schaffung des Integralrotors und durch nur einen einzigen Stator zur Lagerung und zum Antrieb des Integralrotors eine Sonderform des 'lagerlosen Motors' für eine Rotationspumpe darstellt, wird es möglich, äusserst kompakte Rotationspumpen zu bauen.

Bei bei der Verwendung einer solchen elektromagnetischen Rotationsmaschine, z.B. einer Rotationspumpe zum Fördern von Fluiden, die ausserordentlich verschmutzungsanfällig sind, etwa von Blut während einer Operation am offenen Herzen, besteht die Notwendigkeit, nach Gebrauch alle vom Blut berührten Teile perfekt zu säubern bzw. sämtliche Blutreste zu entfernen, bevor die Rotationspumpe für eine weitere Operation verwendet wird; der Grund besteht darin, dass unter allen Umständen vermieden werden muss, dass Blut eines ersten Patienten in den Blutkreislauf von weiteren Patienten gelangt, weil fremdes Blut im allgemeinen zu unerwünschten, sogar lebensgefährdenden Reaktionen führen kann. Da eine einwandfreie Reinigung der Pumpe nicht möglich ist, gibt es zur Vermeidung jeder Gefahr für den Patienten keine andere Möglichkeit, als die Rotationspumpe oder mindestens die vom Blut berührten Teile der Rotationspumpe nach jedem Gebrauch zu ersetzen. Dies bedeutet, dass bei der hohen Anzahl derartiger Operationen die finanziellen Aufwendungen allein für die Rotationspumpe beträchtlich sind. Durch den Einsatz der erfindungsgemässen Rotationspumpe mit dem Integralrotor und dem einzigen Stator lassen sich bei geeigneter konstruktiver Ausführung beträchtliche Einsparungen erzielen, indem man eine Konstruktionsart wählt, bei welcher das Pumpengehäuse mit dem darin aufgenommenen Integralrotor von aussen frei zugänglich und leicht ausbaubar ist. Nur diese beiden Teile werden durch das zu fördernde Fluid berührt und verunreinigt und müssen nach jeder Operation ersetzt werden. Sie werden daher als Wegwerfeinheit gestaltet, während die übrigen Bauteile nicht ausgetauscht werden müssen, sondern für eine grosse Anzahl von Operationen verwendet werden können.

Der Integralrotor, der aus den elektromagnetisch wirksamen Bauteilen des Lager/Antriebs-Rotors und aus dem Rotor der angetriebenen Einrichtung, also etwa dem Pumpenrotor besteht, ist bevorzugt so ausgebildet, dass er eine ringförmige Rotorscheibe aufweist, in welcher die genannten elektromagnetisch wirksamen Bauteile aufgenommen sind und an welcher Rotorschaufeln befestigt sind.

Die erwähnten elektromagnetisch wirksamen Bauteile der Rotorscheibe des Integralrotors umfassen Magnete wie Ringmagnete, Scheibenmagnete oder Schalenmagnete sowie je nach ausgewähltem Typ des Drehfeldmotors Eisen wie Rückschlusseisen oder Eisenkreuze und Wicklungen; sie können in miteinander verschweisste Teile der Rotorscheibe eingebettet oder an der Rotorscheibe mittels einer Spritzmasse so angespritzt sein, dass sie von der Spritzmasse vollständig ummantelt sind.

Damit der Integralrotor und das Rotorgehäuse, welche in gewissen Fällen wie erwähnt als Wegwerfeinheit ausgebildet sind, preisgünstig hergestellt werden können, ist es vorteilhaft, sie so auszubilden, dass alle vom Fluid berührten Teile aus Kunststoff bestehen. Andernfalls müssten kostspielige Materialien verwendet werden, da bei der Auswahl des Materials einerseits darauf zu achten ist, dass keine chemischen Reaktionen zwischen dem Material und dem Fluid ablaufen und anderseits vermieden werden muss, dass die zum Antrieb und zur Steuerung notwendigen elektromagnetischen Felder gestört werden.

Das Pumpengehäuse kann zusätzlich zu dem erwähnten, axialen Einlass einen weiteren, dem ersten gegenüberliegenden Einlass für das Fluid aufweisen. Der Einfluss, den eine entsprechende Konstruktion in Bezug auf die Druckverhältnisse und die erforderlichen Massnahmen zur Steuerung der Position des Integralrotors hat, wird später erläutert.

Entsprechend ist es möglich, das Pumpengehäuse so zu konstruieren, dass es zusätzlich zum erwähnten, mindestens annähernd radialen Auslass einen weiteren Auslass aufweist, der zentralsymmetrisch zum ersten Auslass angeordnet ist. Auch die dadurch entstehenden Einflüsse auf die Druckverhältnisse und die erforderlichen Massnahmen zur Steuerung der Position des Integralrotors werden später erläutert.

Eine Rotationspumpe nach der Erfindung kann als Axialpumpe ausgebildet sein. Dabei werden die Rotorschaufeln durch Flügel eines Flügelrades gebildet, das bevorzugt am inneren Rand der Rotorscheibe des Integralrotors befestigt ist. Meistens wird jedoch eine Zentrifugalpumpe verwendet, da sie die Erzeugung eines höheren Druckes erlaubt. Das Prinzip der kombinierten Lagerung und des Antriebs einer Einrichtung wie z.B. eines Pumpenrotors, lässt sich auch auf alle anderen Arten von Kreiselpumpen wie Seitenkanalpumpen, Peripheralpumpen, Teslapumpen oder Flüssigkeitsringpumpen, Mischeinrichtungen oder auf andere Einrichtungen übertragen, die drehanzutreiben sind.

Bei einem einfachen Typ einer Zentrifugalpumpe sind Rotorschaufeln lediglich an einer Fläche der ringartigen Rotorscheibe des Integralrotors vorgesehen.

Es ist aber auch möglich, an beiden Flächen des Integralrotors Rotorschaufeln anzuordnen, oder die Rotorschaufeln in einem Laufrad zu integrieren.

Der Lager/Antriebsstator kann als axial kurz ausgebildeter, Herkömmlicher Drehstrom-Stator, mit einer Antriebswicklung der Polzahl p und einer Steuerwicklung der Polzahl p+1 oder p-1 ausgeführt sein. Weil bei einer solchen Konstruktion aufgrund der kurzen Länge des Stators im Vergleich zu einem Durchmesser sich nur ein kleiner Teil der Wicklung in der Nut befindet, hat eine solche Ständerkonstruktion erhebliche Nachteile. Ein so konstruierter Ständer, hat nicht nur eine sehr grosse Wickelkopfstreuung, sondern der Wirkungsgrad ist schlecht. Zudem wird es beispielsweise bei einer Zentrifugalpumpe schwierig sein, den Anschluss-Stutzen zwischen den Wickelköpfen hindurch zu führen.

Der Lager/Antriebs-Stator ist im allgemeinen so ausgebildet, dass er mehrere längliche, um den Integralrotor angeordnete Spulenkerne mit einem gemeinsamen magnetischen Rückschluss aufweist. Dabei enthält jeder Spulenkern eine Teilwicklung für jeden Wicklungsstrang der Antriebswicklung mit der Polpaarzahl p und eine Teilwicklung für jeden Wicklungsstrang des Wicklungsstrangs der Steuerwicklung mit der Polpaarzahl p+1 oder p-1. Eine sinus-förmige geometrische Verteilung der Antriebsdurchflutung und der Steuerdurchflutung wird angenähert durch das Verhältnis der Windungszahlen der Teilwicklungen eines Wicklungsstranges. So wird eine Konstruktion erreicht, die mit sehr kurzen Wickelköpfen auskommt. Es ist möglich, dass mindestens eine der Teilwicklungen die Windungszahl null aufweist.

Bei einer üblichen Ausführungsform sind die oben erwähnten Spulenkerne radial zur Rotationsachse des Integralrotors angeordnet. Die Spulenkerne und das Rückschlusseisen können dabei eine Einheit bilden. Diese Einheit kann beispielsweise aus einzelnen Blechen mit langen Nuten geschichtet sein.

Bei einem weiteren bevorzugten Ausführungsbeispiel weisen die Spulenkerne die Form von "L" auf, wobei die einen Schenkel der "L" parallel zur Rotationsachse des Integralrotors angeordnet sind und die anderen Schenkel der "L" radial einwärts zu dieser Rotationsachse gerichtet sind, um den Fluss radial zum Integralrotor zu führen. Ein Drehfeldmotor mit einer solchen Rotor-Geometrie kann als Tempelmotor bezeichnet werden. Der Tempelmotor eignet sich besonders zur Aufnahme einer austauschbaren, als Wegwerfeinheit konzipierten Anordnung aus Pumpengehäuse und Integralrotor.

Der Antriebsteil der Lager/-Antriebs-Vorrichtung kann nach dem Prinzip eines Synchronmotors oder Induktionsmotors ausgebildet sein. Der Synchronmotor führt im allgemeinen zu Konstruktionen mit höherem Wirkungsgrad und vor allem geringeren Rotorverlusten. Im besonderen kann für diesen Synchronmotor eine Konstruktion nach dem Prinzip des Reluktanzmotors oder des permanentmagnetisch erregten Synchronmotors gewählt werden.

Als Teil der Steuer- oder Regeleinrichtung der neuen Rotationspumpe kann, um den Antriebsflusswinkel zu bestimmen, der für die Steuerung des Antriebs des lagerlosen Motors notwendig ist, eine entsprechende Einrichtung vorgesehen sein.

Diese Einrichtung kann beispielsweise eine oder mehrere Flussonden enthalten. Die Rotationspumpe kann im weiteren als Teil ihrer Steuer-oder Regeleinrichtung eine Detektoreinrichtung zur Bestimmung der Position des Integralrotors besitzen. Eine solche Detektoreinrichtung weist im allgemeinen einen X-Y-Detektor auf, mittels welchem die Position des Integralrotors in einer Ebene senkrecht zur Rotationsachse bestimmbar ist. Der X-Y-Detektor kann eine oder mehrere Flussonden enthalten.

Ein bevorzugter X-Y-Detektor weist mehrere symmetrisch verteilte Flussonden auf, um die Teilflüsse an diskreten Stellen zu ermitteln. Aus den Teilflüssen werden zusätzlich zum Antriebsflusswinkel durch den Drehwinkel des Drehfeldmotors die X-Komponente und die Y-Komponente der Position des Integralrotors bestimmt. Dies geschieht durch gewichtete Summation der Teilflüsse über jeweils den halben Umfang in positiver und negativer X-Richtung sowie in positiver und negativer Y-Richtung, durch Betragsbildung der ermittelten Summen und anschliessende Differenzbildung der Anteile in positiver und negativer X-Richtung sowie der Anteile in positiver und negativer Y-Richtung.

Die für die Regelung des Antriebs und/oder die Regelung der Position des Integralrotors in der X-Y-Ebene verwendeten Flussonden können beispielsweise im Luftspalt zwischen dem Pumpengehäuse und dem Lager/Antriebs-Stator und dem Pumpengehäuse angeordnet sein.

Es ist auch möglich, die Flussonden im Kern der länglichen Spulen vorzusehen. Die Flussonden können beispielsweise Hall-Elemente oder magnetoresistive Flussonden sein. Die Befestigung der Flussonden kann an einem Zahn des Stators des Drehfeldmotors, beispielsweise durch Klebung, vorgenommen werden. Eine andere Möglichkeit zur Befestigung der Flussonden besteht darin, sie in Ausnehmungen des Stators einzubetten.

Der X-Y-Detektor kann auch so ausgebildet sein, dass er einen Wirbelstromdetektor enthält um in der senkrecht zur Rotationsachse des Integralrotors verlaufenden X-Y-Ebene den Abstand zu einer im Integralrotor vorhandenen leitfähigen Schicht zu messen. Diese leitfähige Schicht kann beispielsweise aus einem Metallring oder einer dünnen Metallschicht oder aus dem aus leitfähigem Material wie NdFe gefertigten Rotormagneten selbst gebildet sein. Anstelle eines einzigen Wirbelstromdetektors können auch in X- und Y-Richtung je zwei einander gegenüberliegende Wirbelstromdetektoren angeordnet sein, welche Detektorsignale abgeben. Auf diese Weise lassen sich die X- und die Y-Komponente der Position des Integralrotors in der quer zur Rotationsachse verlaufenden X-Y-Ebene bestimmen.

Der X-Y-Detektor kann auch so ausgebildet sein, dass er Sensorwicklungen enthält, welche zusätzlich zur Antriebswicklung und zur Steuerwicklung im Stator des Drehfeldmotors angeordnet sind, um die Position des Integralrotors in der quer zu seiner Rotationsachse verlaufenden X-Y-Ebene mittels Auswertung der elektrischen Impedanz dieser Sensorwicklungen zu bestimmen.

Eine weitere Möglichkeit, den X-Y-Detektor auszubilden, besteht darin, eine optische Einrichtung vorzusehen, durch welche die Position des Integralrotors in der quer zu seiner Rotationsachse verlaufenden X-Y-Ebene mit Hilfe von Licht gemessen werden kann, dessen Wellenlänge im optischen Fenster des Fluids liegt.

Die erwähnte Detektoreinrichtung kann neben dem X-Y-Detektor, von welchem verschiedene Varianten ausführlich dargelegt worden sind, auch einen Z-Detektor besitzen, um die axiale Position des Integralrotors zu erfassen und ein entsprechendes Z-Positionssignal zu emittieren.

Dabei kann das ermittelte Z-Positionssignal den Ist-Wert für die Bestimmung eines Regelsignals für eine regeltechnische Stabilisierung der axialen Position des Integralrotors bilden. Zu einer solchen regeltechnischen Stabilisierung der axialen Position des Integralrotors kann auch ein Magnetisierungsstrom mit einer Stromkomponente in der Antriebsflussrichtung des Lager/Antriebsektors aufgebracht werden.

Die Rotationspumpe weist vorzugsweise eine Druckbestimmungsvorrichtung auf, mit welcher aus dem Z-Positionssignal oder aus dem daraus ermittelten Regelsignal der Pumpendruck bestimmbar ist.

Im allgemeinen ist die Rotationspumpe auch mit einer Durchflussbestimmungsvorrichtung ausgerüstet, welche aus dem Z-Positionssignal sowie der Drehzahl des Integralrotors und der drehmomentbildenden Komponente des Antriebsstroms den zeitlichen Durchfluss des Fluids bestimmt.

Im folgenden wird die Erfindung und werden weitere Eigenschaften und Vorteile der erfindungsgemässen Rotationsmaschine anhand schematischer Zeichnungen von Ausführungsbeispielen wie Rotationspumpen und Rührwerken sowie Einzelheiten davon beschrieben. Es zeigen:
- Fig. 1: eine Rotationspumpe nach der Erfindung in einer schematischen Schnitt-Darstellung durch die Rotationsachse;
- Fig. 2: ein Pumpengehäuse mit einem Integralrotor, in einer ersten Ausführungsform, in einem Schaubild;
- Fig. 3: ein Pumpengehäuse mit einem Integralrotor, in einer zweiten Ausführungsform, in einem Schaubild;
- Fig. 4: eine Rotationspumpe mit zusätzlicher Axialwicklung, in stark vereinfachter Darstellung, in einem Schnitt längs der Rotationsachse; (nicht Gegenstand der Erfindung)
- Fig. 5: eine weitere Rotationspumpe mit zwei Axialwicklungen, in gleicher Darstellung wie Fig. 4, in vereinfachter Darstellung, in einem Schnitt längs der Rotationsachse; (nicht Gegenstand der Erfindung)
- Fig. 6: einen Integralrotor für eine Axialpumpe, in einem Schaubild;
- Fig. 7: einen Integralrotor für eine Zentrifugalpumpe, in einem Schaubild;
- Fig. 8: einen Integralrotor für eine Zentrifugalpumpe, zur Verwendung mit einer nach dem Prinzip des Reluktanzmotors arbeitenden Antriebsvorrichtung, in einem Schaubild;
- Fig. 9: eine als Axialpumpe ausgebildete Rotationspumpe in vereinfachter Darstellung, in einem Schaubild;
- Fig. 10: eine als Zentrifugalpumpe ausgebildete Rotationspumpe in vereinfachter Darstellung, in einem Schaubild;
- Fig. 11: einen Lager/Antriebs-Stator mit einem Integralrotor, wobei zur Vereinfachung das Pumpengehäuse weggelassen ist, in einem Schaubild;
- Fig. 12: eine Rotationspumpe mit einer als Tempelmotor ausgebildeten Lager/Antriebs-Vorrichtung, in einem Schaubild;
- Fig. 13: einen Lager/Antriebs-Stator mit einem Integralrotor, in stark vereinfachter Darstellung, in einem ersten Betriebszustand, von oben und mit Flussensoren für das Messen des Winkels;
- Fig. 14: den Lager/Antriebs-Stator mit dem Integralrotor der Fig. 11 in gleicher Darstellung wie in Fig. 13, jedoch mit einer anderen Position des Integralrotors und mit Flussensoren zur Bestimmung der Position des Rotors in der X-Y-Ebene;
- Fig. 15: einen weiteren Lager/Antriebs-Stator mit einem Integralrotor, in stark vereinfachter Darstellung, von oben, wobei eine mögliche Anordnung von Sensorwicklungen gezeigt ist;
- Fig. 16: ein Diagramm zur Verdeutlichung der Linearität (Arbeitsbereich) des Positionssignals U₁-U₂, welches man durch Differenzbildung der Signale U₁ und U₂ von zwei gegenüberliegenden Poitionssensoren erhält, bzw. Darstellung der resultierenden Spannung U in Abhängigkeit von der Position des Integralrotors in X-Richtung;
- Fig. 17: einen Stator, bei dem die Windungszahlen der Drehfeldwicklung und der Steuerwicklung so gewählt sind, dass eine sinusförmige geometrische Verteilung der Antriebsfeld-Durchflutung und der Steuerfeld-Durchflutung erreicht wird;
- Fig. 18: eine Teslapumpe mit seitlich in den Tesla-Pumpenrotor integriertem, antreibenden Rotor;
- Fig. 19: eine Teslapumpe mit einem in das Innere des Tesla-Pumpenrotors integrierten antreibenden Rotor;
- Fig. 20: ein Spaltrohr-Rührwerk mit einem magnetgelagerten Integral-Rühr-Rotor in einem Spaltrohr;
- Fig. 21: ein Spaltrohr-Rührwerk mit einem magnetgelagerten Integral-Rühr-Rotor in einem Spaltrohr mit einem elektromagnetischen Axial-Lager für den Integralrotor; (nicht Gegenstand der Erfindung)
- Fig. 22: eine weitere vorteilhafte Ausführungsform einer Lager/Antriebsvorrichtung mit Permanentmagneten im Stator.

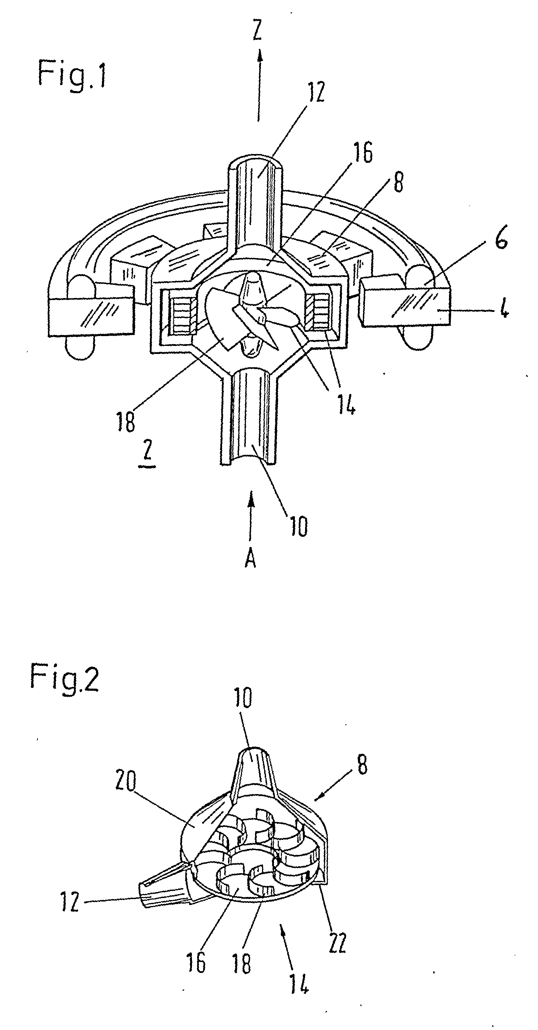

Die in Fig. 1 dargestellte Rotationspumpe 2 weist einen Lager/Antriebs-Stator 4 und eine nach dem Prinzip des 'lagerlosen Motors' wirkenden Lager/Antriebsvorrichtung auf. Der lagerlose Motor arbeitet nach dem Prinzip eines Drehfeldmotors. Der Lager/Antriebs-Stator 4 besitzt elektromagnetisch wirksame Bauteile 6, die später ausführlich beschrieben werden. Im Lager/Antriebs-Stator 4 ist ein Pumpengehäuse 8 mit einem axialen Einlass 10 und einem axialen Auslass 12 für ein zu förderndes Fluid angeordnet (Axialpumpe). Der Einlass 10 und der Auslass 12 dienen dazu, die Rotationspumpe 2 mit einem nicht dargestellten Leitungsnetz zu verbinden. Ein erster Pfeil A bezeichnet die Zuflussrichtung, ein zweiter Pfeil Z die Abflussrichtung des Fluids. Der Lager/Antriebs-Stator 4 und das Pumpengehäuse 8 sind ortsfeste bzw. beim Pumpenbetrieb nicht drehende Bauteile der Rotationspumpe 2. Im Inneren des Pumpengehäuses 8 befindet sich ein Integralrotor 14, der durch eine Rotorscheibe 16 und daran befestigte Rotorschaufeln 18 gebildet wird. Der Integralrotor 14 dient gleichzeitig als Lager/Antriebs-Rotor wie auch als Pumpenrotor und ist somit das einzige drehbare Bauteil der Rotationspumpe 2.

Das in Fig. 2 dargestellte Pumpengehäuse 8 besitzt einen axialen Einlass 10 und einen radialen Auslass 12, eine konische obere Wandung 20 und eine im wesentlichen ebene untere Wandung 22. Im Pumpengehäuse 8 ist der Integralrotor 14 sichtbar mit der hier ringförmig ausgebildeten Rotorscheibe 16 und den Rotorschaufeln 18, die an der oberen Fläche der Rotorscheibe 16 befestigt sind. Ein solcher Integralrotor wird verwendet in einer als Zentrifugalpumpe ausgebildeten Rotationspumpe.

Es wurde eingangs schon erwähnt, dass die Anzahl und damit die konstruktive Anordnung der Einlässe und Auslässe die Druckverhältnisse und damit die Lage des Integralrotors im Pumpengehäuse beeinflussen. Um diesen Einfluss in der X-Y-Ebene zu minimalisieren, ist es bei einer Ausbildung der Rotationspumpe als Zentrifugalpumpe vorteilhaft, zwei sich gegenüberliegende Auslässe vorzusehen. In diesem Zusammenhang wäre es sogar vorteilhaft, eine Vielzahl von Auslässen vorzusehen, was aber die Konstruktion stark komplizieren würde.

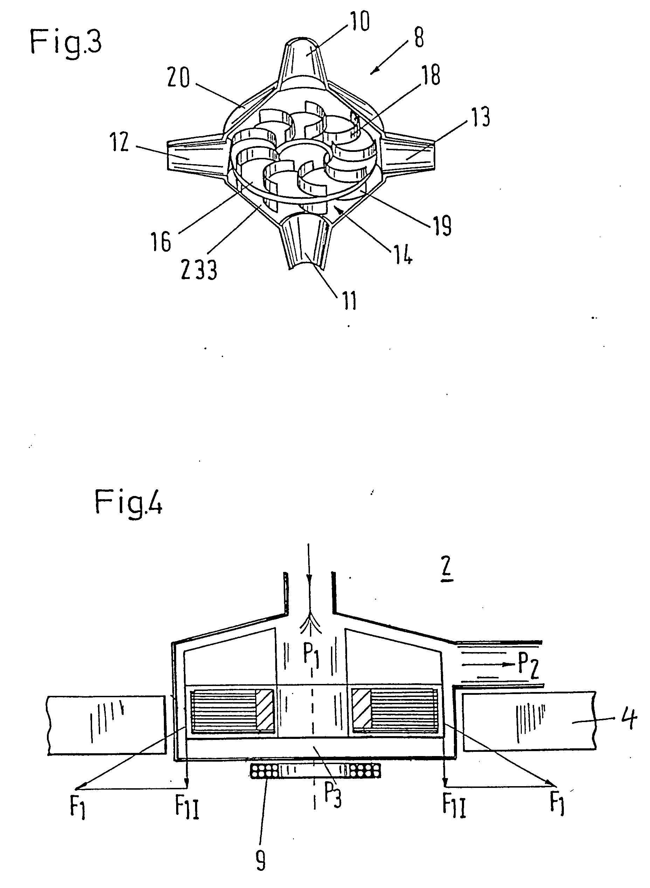

Das in Fig. 3 gezeigte Pumpengehäuse 8 weist einen weiteren axialen Einlass 11 und einen weiteren radialen Auslass 13 auf, wobei sich sowohl die Einlässe 10, 11 als auch die Auslässe 12, 13 jeweils gegenüberliegen. Das Pumpengehäuse 8 besitzt daher neben der oberen konischen Wandung 20 auch eine untere konische Wandung 233, und die Rotorscheibe 16 des Integralrotors 14 ist auf ihrer oberen und ihrer unteren Fläche mit Rotorschaufeln 18 bzw. 19 bestückt.

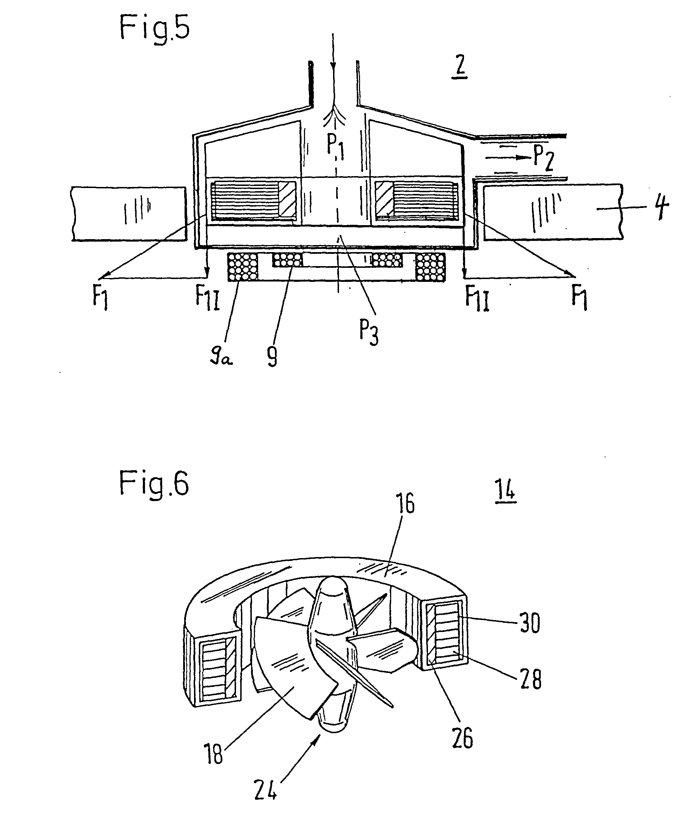

Bei einer Ausbildung der Rotationspumpe als Axialpumpe sowie als Zentrifugalpumpe mit nur einem axialen Einlass wirkt infolge des Druckgefälles in axialer Richtung eine axiale Kraft auf den Integralrotor. Fig. 4 und Fig. 5 zeigen am Beispiel einer Zentrifugalpumpe, wie diese axiale Kraft kompensiert werden kann, indem eine oder mehrere zusätzliche Axialwicklungen 9 bzw. 9a angeordnet werden; dies ist jedoch nicht Gegenstand der Erfindung.

Der Integralrotor 14 gemäss Fig. 6 ist für eine nach dem Prinzip einer Axialpumpe wirkende Rotationspumpe bestimmt. Er besteht im wesentlichen aus der ringförmigen Rotorscheibe 16 und einem im zentralen Freiraum dieser Rotorscheibe 16 angeordneten Flügelrad 24, dessen Flügel die Rotorschaufeln 18 bilden. Die aufgeschnitten dargestellte Rotorscheibe 16 enthält in ihrem Inneren elektromagnetisch wirksame Bauteile für eine Lager/Antriebs-Vorrichtung mit einem als Synchronrotor wirkenden Drehfeldmotor, nämlich ein rotorseitiges Rückschlusseisen 26, einen Magneten 28 und eine elektrisch leitende Schicht 30; diese Schicht 30 kann aus einem Metallring oder einer dünnen Metallschicht bestehen. Ist der Magnet 28 aus elektrisch leitfähigem Material gefertigt, so kann er selbst als leitfähige Schicht benutzt werden. Sie wird als Messeinheit für die Messung der radialen Rotorposition mittels Wirbelstromdistanzsensoren genutzt.

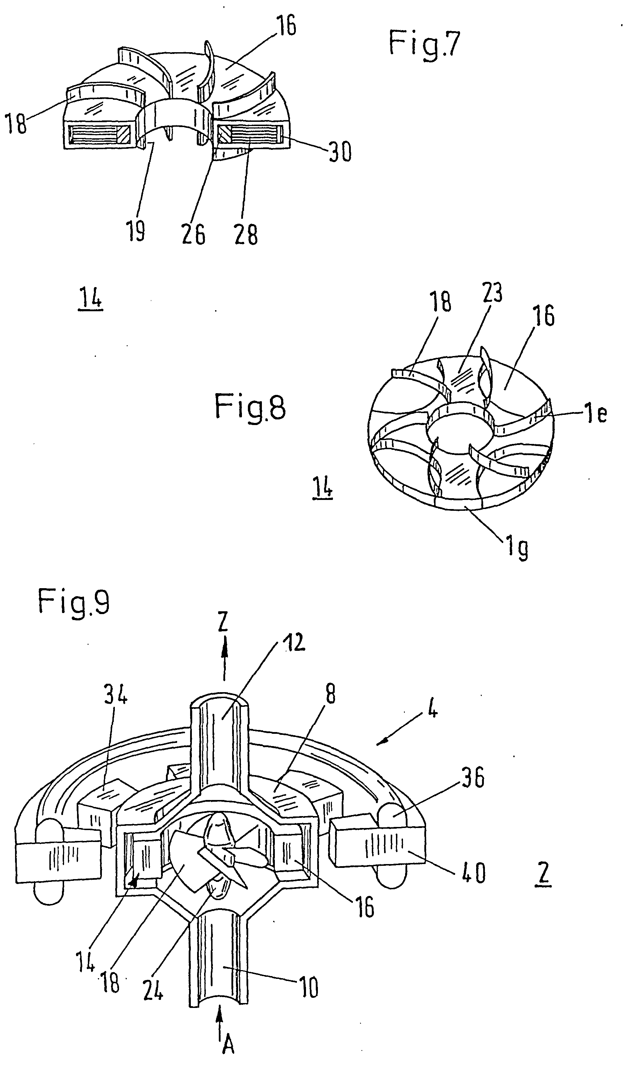

Fig. 7 zeigt nochmals den in Fig. 3 dargestellten, in einer Zentrifugalpumpe oder Seitenkanalpumpe verwendbaren Integralrotor 14, mit den oberen Rotorschaufeln 18 und den unteren Rotorschaufeln 19 sowie mit der hier aufgeschnittenen Rotorscheibe 16, in welcher das rotorseitige Rückschlusseisen 26, der Magnet 28 und die leitende Schicht 30 eingeschlossen sind, die als rotierende Teile eines Synchronmotors zu betrachten sind.

Der in Fig. 8 dargestellte Integralrotor 14 mit der ringförmigen Rotorscheibe 16 ist für eine als Zentrifugalpumpe konzipierte Rotationspumpe bestimmt, deren Lager/Antriebs-Vorrichtung einen Drehfeldmotor besitzt, der nach dem Prinzip eines Reluktanzmotors arbeitet. Nur die obere Fläche der Rotorscheibe 16 ist mit den Rotorschaufeln 18 bestückt. In der Rotorscheibe ist ein Eisenkreuz 23 eingebettet, wie es für einen Reluktanzmotor üblich ist.

Fig. 9 zeigt die als Axialpumpe ausgebildete Rotationspumpe 2 deutlicher. Das aufgeschnittene Pumpengehäuse 8 weist den axialen Einlass 10 und den hier ebenfalls axialen Auslass 12 auf, wobei die Pfeile A und Z die Richtung des geförderten Fluids anzeigen. Im Pumpengehäuse 8 befindet sich der Integralrotor 14, der wie früher beschrieben durch die Rotorscheibe 16 und das Flügelrad 24 mit seinen als Rotorschaufeln 18 wirkenden Flügeln gebildet wird. Im weiteren ist in Fig. 9 der Lager/Antriebs-Stator 4 sichtbar. Er besteht aus dem Statorblechpaket 40 mit Nuten, Zähnen und Rückschlusseisen und einer in die Statornuten eingelegte Wicklung 36, welche aus einer Teilwicklung mit der Polzahl p und einer Teilwicklung mit der Polzahl p+1 oder p-1 besteht.

Mehrere symmetrisch um das Pumpengehäuse 8 angeordnete, radial gerichtete stabartige Spulenkerne 34, Wicklungen 36 und ein statorseitiges Rückschlusseisen 38 bilden die ortsfesten elektromagnetisch wirksamen Bauteile der Lager/Antriebs-Vorrichtung der Rotationspumpe 2.

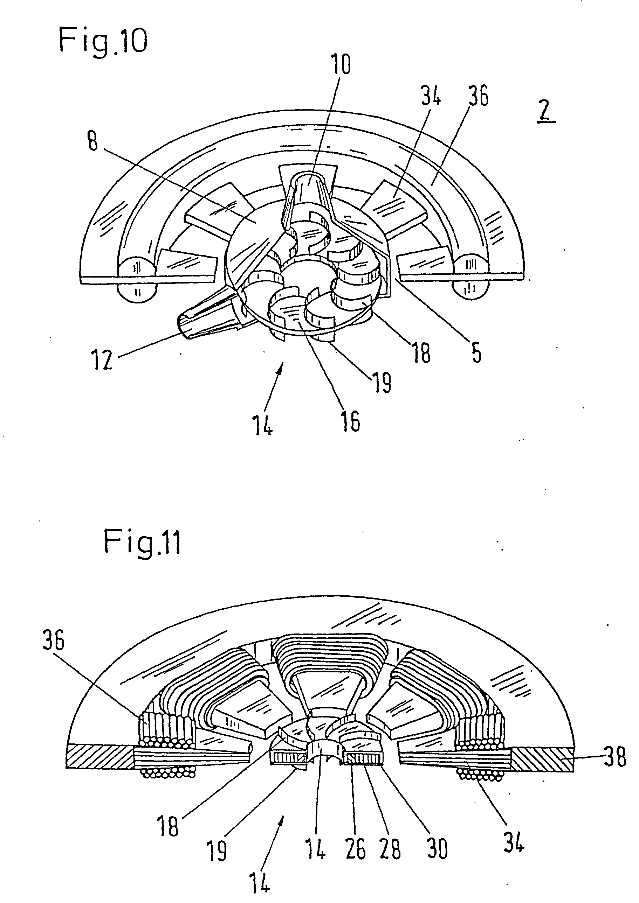

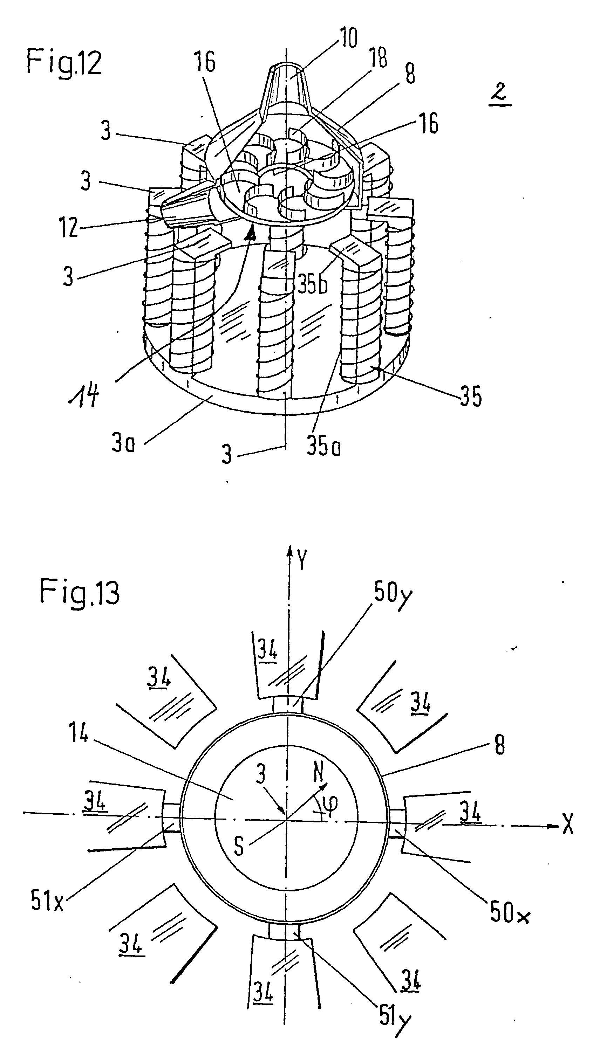

In Fig. 10 ist eine Ausführungsform dargestellt, bei welcher die Rotationspumpe 2 als Zentrifugalpumpe ausgebildet ist. Man erkennt das Pumpengehäuse 8 mit dem axialen Einlass 10 und dem hier radialen Auslass 12 sowie den Integralrotor 14 mit der ringförmigen Rotorscheibe 16 und den Rotorschaufeln 18. Im weiteren sind die radial gerichteten Spulenkerne 34, die Wicklungen 36 und das Rückschlusseisen 38 (Fig. 11) vereinfacht dargestellt.

Fig. 11 zeigt die rotorseitigen und statorseitigen elektromagnetisch wirksamen Bauteile deutlicher. Dabei wurde das Pumpengehäuse als elektromagnetisch unwirksames Bauteil weggelassen. In der Mitte befindet sich der aufgeschnittene Integralrotor 14 mit der Rotorscheibe 16, welche mit den Rotorschaufeln 18, 19 versehen ist. In der Rotorscheibe 16 sind, wie weiter oben beschrieben, das Rückschlusseisen 26, der Magnet 28 und die leitende Schicht 30 eingebettet. Der Integralrotor 14 ist vom ebenfalls aufgeschnittenen Lager/Antriebs-Stator umgeben, welcher die Spulenkerne 34, die Wicklungen 36 und das rotorseitige Rückschlusseisen 38 aufweist.

In Fig. 12 ist die Rotationspumpe 2 mit einer Lager/Antriebsvorrichtung dargestellt, deren Drehfeldmotor ein sogenannter Tempelmotor ist. Zur Verdeutlichung dieser Figur ist das Pumpengehäuse 8 nicht in seiner montierten Lage sondern oberhalb derselben dargestellt. Wie schon mehrfach beschrieben, weist das Pumpengehäuse 8 einen axialen Einlass 10 und einen radialen Auslass 12 auf. Es enthält den Integralrotor 14 mit der ringförmigen Rotorscheibe 16, an deren oberer Fläche die Rotorschaufeln 18 sichtbar sind. Anstelle der radial verlaufenden Spulenkerne 34, wie sie in den Fig. 9,10 und 11 dargestellt sind, weist der Tempelmotor symmetrisch verteilte Spulenkerne 35 auf, von denen jeder die Form eines 'L' hat. Dabei verläuft der eine lange Schenkel 35a des 'L' vertikal. bzw. parallel zur Rotationsachse 3, während der kurze Schenkel 35b des 'L' radial einwärts zur Rotationsachse 3 gerichtet ist. Der Tempelmotor unterscheidet sich in seiner elektrischen Wirkungsweise daher nicht von dem in Fig. 11 dargestellten Drehfeldmotor, erlaubt es aber besonders gut, das Pumpengehäuse 8 praktisch im Lager/Antriebs-Stator so anzuordnen, dass es zwar im montierten Zustand praktisch versenkt und somit platzsparend angeordnet ist, aber dennoch in einfacher Weise demontierbar ist. Im weiteren sind die Wicklungen 36 sowie das Rückschlusseisen 38 sichtbar.

Die obige Beschreibung bezieht sich im wesentlichen auf den konstruktiven Aufbau der Rotationspumpe mit ihrer Lager/Antriebs-Vorrichtung und der Steuerung. Im folgenden werden mögliche Massnahmen zur regeltechnischen Ausbildung der Rotationspumpe dargelegt.

Um den für die Antriebsregelung des lagerlosen Motors notwendigen Antriebsflusswinkel zu bestimmen, kann mindestens eine Flussonde vorgesehen werden. Über die Teilflusskomponente ist dann der Antriebsflusswinkel bestimmbar. Über dieselbe Anordnung ist ebenfalls die Drehzahl des Rotors bestimmbar. Die Anordnung und Ausbildung der Flussonden wird später beschrieben.

Zur regeltechnischen Stabilisierung der Position des Integralrotors muss dessen jeweilige Position bzw. die Abweichung derselben von der soll-Position ermittelt werden. Dazu ist eine Detektorvorrichtung vorgesehen, welche einen X-Y-Detektor und auch einen Z-Detektor aufweist. Der X-Y-Detektor dient zur Bestimmung der Position des Integralrotors in der senkrecht zur Rotationsachse verlaufenden X-Y-Ebene, und der Z-Detektor dient zur Bestimmung der Position des Integralrotors längs der Rotationsachse des Integralrotors.

Der X-Y-Detektor kann in einer ersten Ausführungsart mehrere Flussonden aufweisen, wie sie auch für die Regelung des Antriebes benützt werden können. Dabei können die Flussonden Hall-Elemente oder magnetoresistive Flussonden sein; sie werden beispielsweise an einem Zahn des Stators befestigt, evtl. durch Klebung, oder in eine Nute des Stators eingebettet.

Fig. 13 zeigt die Kontur des Pumpengehäuses 8 und den darin befindlichen Integralrotor 14, der seine korrekte, mittige Position einnimmt. Die als Punkt sichtbare Rotationsachse 3 bildet den Schnittpunkt eines Achsenkreuzes mit den Achsen X und Y. Das Pumpengehäuse 8 ist von den Spulenkernen 34 oder Statorzähnen umgeben. Im Luftspalt zwischen dem Pumpengehäuse 8 und den Spulenkernen 34 oder den Statorzähnen sind mehrere Flussonden 50x, 50y, 51x, 51y angeordnet, welche Detektoren Teil einer Detektoranlage zur Bestimmung der Magnetisierungsrichtung (Winkel ϕ des Integralrotors 14 in der durch die Achsen X und Y definierten, senkrecht zur Rotationsachse 3 verlaufenden X-Y-Ebene sind; diese Lage wird kurz als Winkellage des Integralrotors 14 bezeichnet. Durch die Anordnung von jeweils zwei gegenüberliegenden Flussonden kann der Einfluss einer Verlagerung des Rotors aus dem Zentrum auf die Bestimmung der Winkellage des Integralrotors kompensiert werden.

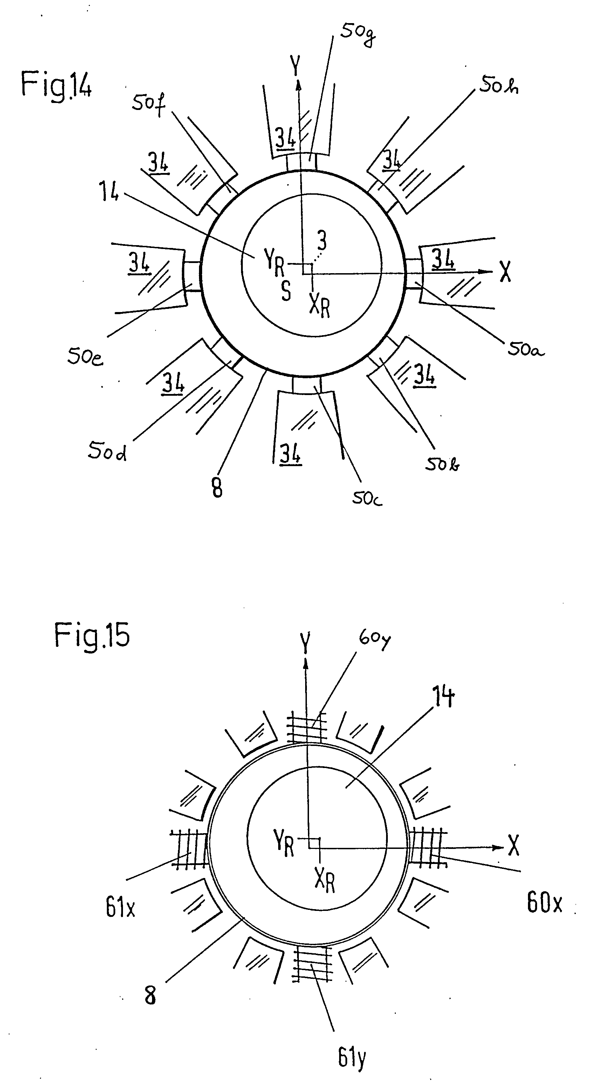

In Fig. 14 ist eine ähnliche Anordnung dargestellt, mit dem Pumpengehäuse 8 und dem darin befindlichen Integralrotor 14, der hier nicht seine korrekte, mittige Lage einnimmt sondern - auf den Achsen X und Y gemessen - um X_{R} und Y_{R} aus dieser Lage verschoben ist, so dass seine Rotationsachse nicht mit dem Schnittpunkt der Achsen X, Y zusammenfällt. Bei dieser Anordnung befinden sich acht Flussonden 50a bis 50h im Luftspalt zwischen dem Rotorgehäuse 8 und jeder der acht ebenfalls eingezeichneten Spulen 34.

Die Flussonden dienen hier nicht nur der Bestimmung der Winkellage des Integralrotors, sondern gleichzeitig der Bestimmung seiner X-Y-Position. Dies geschieht durch gewichtete Summation der mit Hilfe der Flussonden gemessenen Teilflüsse über jeweils den halben Umfang in X-Richtung und Y-Richtung und jeweils in der Gegenrichtung, Betragsbildung und anschliessende Differenzbildung (des Anteils in X-Richtung und in Gegenrichtung sowie des Anteils in Y-Richtung und in Gegenrichtung).

Der X-Y-Detektor kann in einer anderen Ausführungsart auch als Wirbelstrom-Distanzsensor ausgebildet sein. Durch einen solchen Wirbelstrom-Distanzsensor wird der Abstand zu der mehrfach erwähnten leitfähigen Schicht in der Rotorscheibe des Integralrotors in der X-Y-Ebene gemessen.

In einer anderen Ausführungsart gemäss Fig. 15 enthält der X-Y-Detektor Sensorwicklungen 60x, 60y, 61x, 61y. Die übrigen in Fig. 15 dargestellten Elemente entsprechen denen der Fig. 14. Die Sensorwicklungen bestimmen die X-Y-Position des Integralrotors 14 durch Auswertung ihrer elektrischen Impedanz.

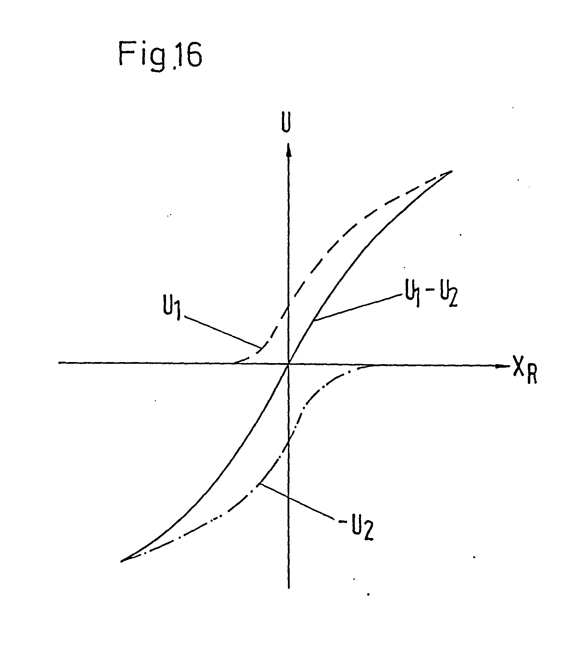

Fig. 16 zeigt den Zusammenhang zwischen Spannung U und Abweichung X_{R} des Integralrotors von seiner soll-Lage in Richtung der Achse X. Daraus ist ersichtlich, dass der Zusammenhang U/x im mittleren, für die Regelung massgebenden Bereich in erwünschter Weise linear ist, falls das Sensorsignal aus der Differenz U₁-U₂ von zwei gegenüberliegenden Sensoren mit eingeschränktem, linearem Messbereich gewonnen werden

Eine weitere Ausführungsform für die Ausbildung des X-Y-Detektors besteht darin, eine optische Einrichtung zu verwenden, wobei Licht, dessen Wellenlänge im Bereich des optischen Fensters des geförderten Fluids liegt, verwendet wird.

Die Detektoreinrichtung weist, wie schon erwähnt, nicht nur den X-Y-Detektor sondern eventuell auch einen Z-Detektor auf, für welchen übliche Detektoreinrichtungen, beispielsweise Wirbelstrom-Distanzsensoren benutzt werden. Der X-Y-Detektor bestimmt die Position des Integralrotors in Richtung der Rotationsachse und emittiert ein Z-Positionssignal, welches als ist-Wert für die regeltechnische Stabilisierung des Integralrotors in dieser Richtung benützt wird. Zu dieser regeltechnischen Stabilisierung dient beispielsweise ein Magnetisierungsstrom, der in der Antriebsrichtung mit einer Stromkomponente in der Flussrichtung aufbringbar ist. Das erwähnte Z-Positionssignal oder das daraus ermittelte Regelsignal dient auch dazu, den Pumpendruck mit Hilfe einer Druckbestimmungsvorrichtung zu bestimmen.

Ebenfalls aus dem Z-Positionssignal lässt sich, bei Kenntnis der Drehzahl des Integralrotors und der drehmomentbildenden Komponente des Antriebsstroms, die zeitlich geförderte Menge des Fluids bestimmen.

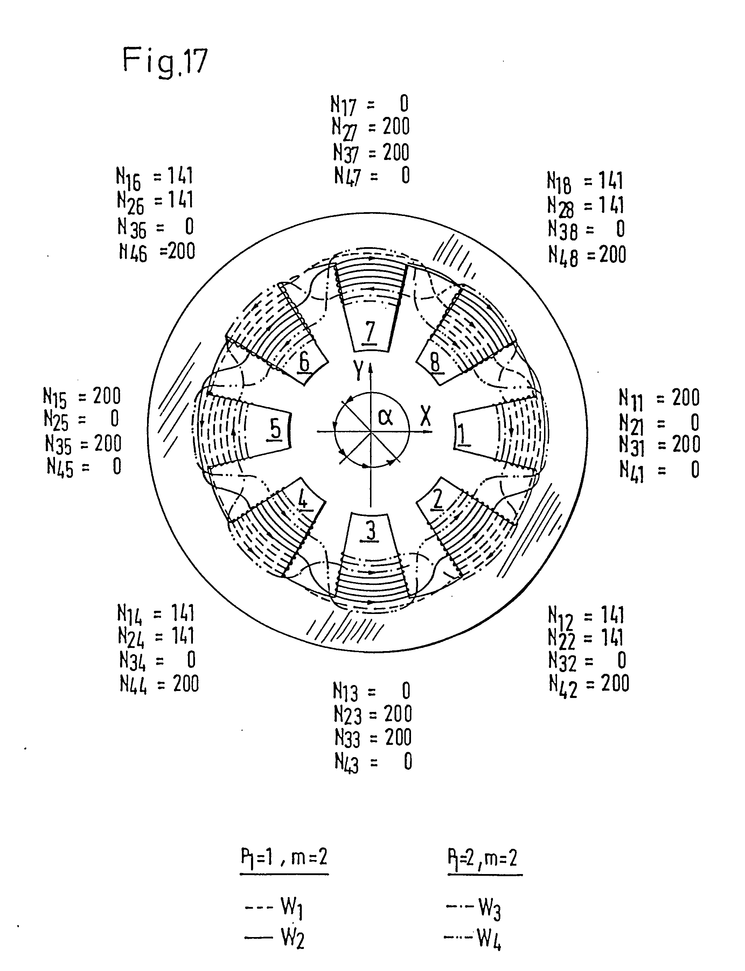

In Fig. 17 ist ein Beispiel für die Windungszahlen der Drehfeldwicklung und der Steuerwicklung gezeigt, mit welchen eine wenigstens angenähert sinusförmige Antriebsdurchflutung und Steuerdurchflutung erreicht wird. Die Zahl der Windungen ist abhängig vom Kosinus bzw. Sinus des elektrischen Winkels α der Lage der Pole.

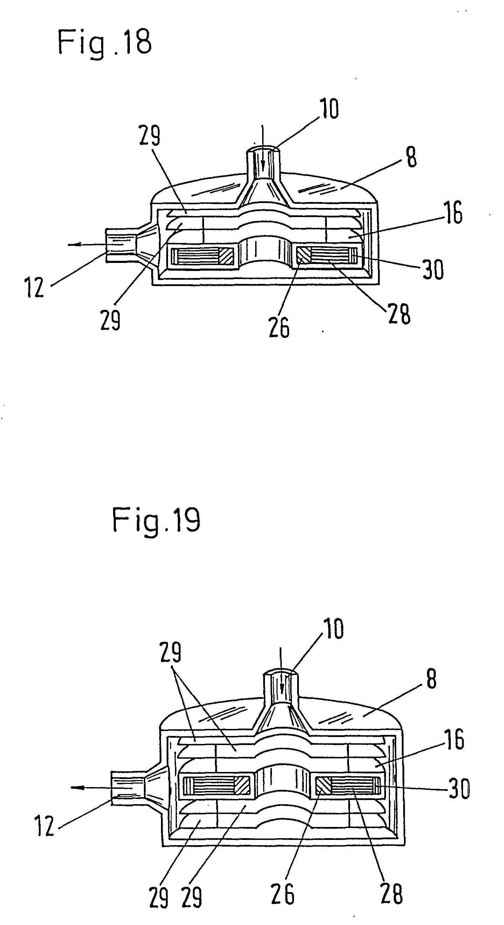

Bei der in Fig. 18 dargestellten Teslapumpe besteht der scheibenförmige, antreibende Rotor aus der Rotorscheibe 16, dem Magneten 28, dem Rückschlusseisen 26, sowie der leitenden Schicht 30. Dieser antreibende Rotor 16 ist mit den Pumpenrotorplatten 29 zu einem Integral-Teslapumpenrotor zusammengebaut. Der antreibende Rotor ist seitlich im Integralrotor angeordnet und ist im Pumpengehäuse 8 mit dem Einlass 10 und dem Auslass 12, eingebaut.

Im Unterschied zur Konstruktion von Fig. 18 ist beim Integralrotor der Teslapumpe von Fig. 19, der antreibende Rotor 16 zwischen Pumpenrotorplatten 29 eingebaut.

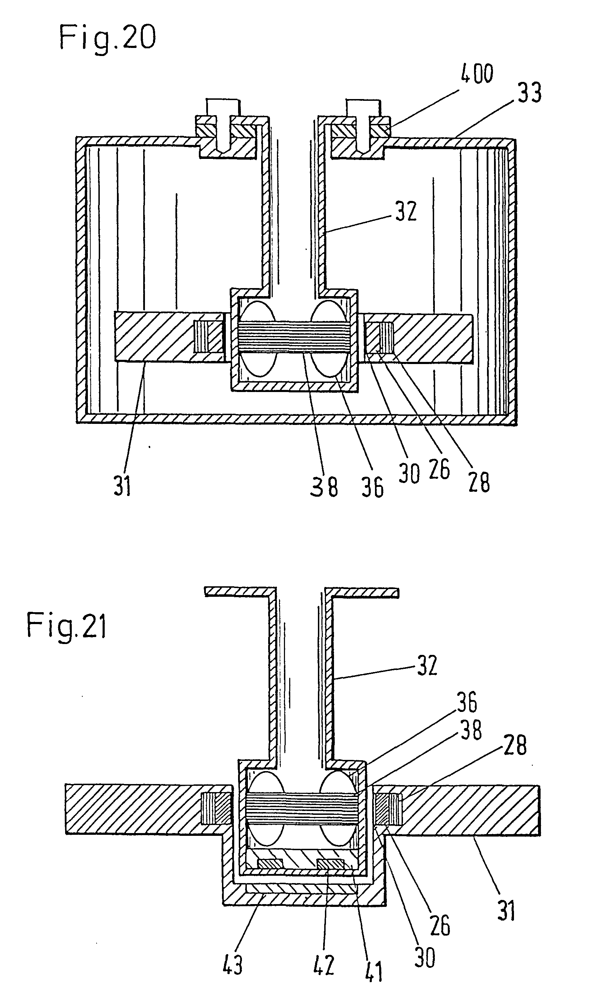

Beim Spaltrohr-Rührwerk von Fig. 20 ist ein Stator mit dem Rückschlusseisen 38 und den Wicklungen 36 im Spaltrohrgehäuse 32 eingebaut. Der Integralrotor 31 umfasst den eigentlichen Rührer, in den der antreibende Rotor mit Magnetring 28, dem Rückschlusseisen 26 und einer leitenden Schicht 30 integriert ist. Das Spaltrohrgehäuse 32 ist in einer Öffnung im Rührtank 33 eingeführt und kann mit der Dichtung 400 gegen den Aussenraum hin abgedichtet sein. Das umzurührende Rührgut befindet sich im Innern des Rührtanks 33.

Beim Spaltrohr-Rührwerk der Fig. 21 (nicht Gegenstand der Erfindung) ist im Spaltrohr 32 zusätzlich ein elektromagnetisches Axiallager mit dem Stator 41 und mit einer Statorwicklung 42 eingebaut. Das Rückschlusseisen 43 des Axialmagnetlagers ist ebenfalls im Rührer 31 integriert.

Der Vorteil einer Lager/Antriebs-Vorrichtung nach dem Prinzip des Reluktanzmotors (Fig. 8) oder Induktionsmotors liegt im Vergleich mit einem permanentmagnetisch erregten Synchronmotor darin, dass der Integralrotor keine teuren Materialien enthält. Dies ist von besonderer Bedeutung, falls der Integralrotor zu einem Wegwerfteil wie beispielsweise bei einer Einweg-Blutpumpe gehört. Nachteilig ist sowohl beim Reluktanzmotor als auch beim Induktionsmotor, dass die gesamte Durchflutung zur Erregung des Antriebsfeldes über einen Magnetisierungsstromanteil in der Antriebswicklung aufgebracht werden muss. Bei grossen Luftspalten erfordert die Luftspaltmagnetisierung enorm hohe Magnetisierungsströme, welche ihrerseits grosse Verluste in der Antriebswicklung verursachen. So ist der mit Induktions- und Reluktanzmotoren erreichbare Luftspalt auf wenige mm beschränkt. Eine Ausnahme bilden sehr grosse Motoren.

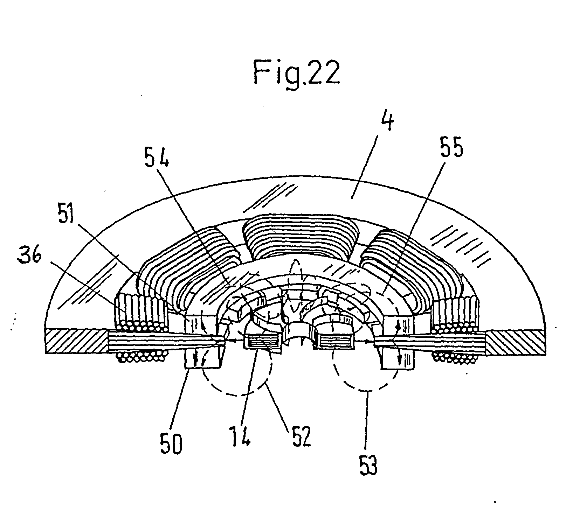

Eine Lösung diesen Problems wird darin gefunden, dass im Stator der Lager/Antriebs-Vorrichtung Permanentmagnete angeordnet werden, welche einen Unipolarfluss erzeugen (das heisst der Fluss wird über den ganzen Umfang betrachtet entweder nur vom Stator zum Rotor oder in umgekehrter Richtung geleitet). Zusammen mit einer zweipoligen (also einpolpaarigen) Drehfeldwicklung lassen sich auf den Integralrotor Kräfte in radialer Richtung erzeugen. Mit einer weiteren Drehfeldwicklung mit einer Polpaarzahl p≥2 kann zusätzlich eine rotierende Drehfeldkomponente erzeugt werden, welche sich zum Antreiben des als Kurzschlussläufers oder als Reluktanzmotorläufers ausgestalteten Integralrotors eignet und weder im Zusammenspiel mit dem Unipolarfluss noch mit der zweipoligen (einpolpaarigen) Flusskomponente zu störenden Radialkräften führt. Eine vorteilhafte Ausführungsform einer solchen Rotationsmaschine ist in Figur 22 dargestellt. Sie zeigt den Stator 4 und den Integralrotor 14 der Lager/Antriebs-Vorrichtung. Der Unipolarfluss, dargestellt durch die Unipolarflusslinien 52,53,54,55, sowie durch weitere Unipolarflusslinien, welche nicht benannt sind, wird hier durch zwei ringförmig ausgeführte lateral magnetisierte und beidseitig des Stators angeordnete Permanentmagnete 50, 51 erregt. Natürlich können an Stelle der Permanentmagnetringe auch mehrere kleine Einzelmagnete eingesetzt werden. Weiter können die Magnete auch ausserhalb der Wicklung angeordnet werden. Die beidseitige Anordnung von Magneten ist deshalb vorteilhaft, weil dadurch die Streufeldlinien welche axial in den Rotor eindringen symmetrisch sind und somit keine axialen Zugkräfte erzeugen. Die Statorwicklung 36 enthält sowohl Windungen einer zweipoligen (einpolpaarigen) Drehfeldwicklung als auch einer höherpoligen Drehfeldwicklung, vorzugsweise mit der Polpaarzahl drei.

Generell lässt sich folgendes sagen:
Die Rotationsmaschine mit einem angetriebenen Rotor und mit einem elektrischen Motor umfasst einen Stator und einen antreibenden Rotor. Der Stator ist auch als elektromagnetisches Lager für den antreibenden Rotor ausgebildet und der antreibende Rotor des Elektromotors bildet mit dem angetriebenen Rotor der Rotationsmaschine eine Rotoreinheit, d.h. einen Integralrotor. Es ist insgesamt nur ein einziger Stator vorgesehen, wobei dieser einzige Stator den Integralrotor sowohl lagert als auch antreibt, und wobei die axiale Länge des antreibenden Rotors kleiner oder gleich dem halben Durchmesser dieses Rotors ist und des Integralrotor axial sowie gegen Verkippung gegenüber der Statorebene passiv durch Reluktanzkräfte stabilisiert ist.

Eine solche Rotationsmaschine umfasst typischerweise eine Antriebs-/Drehfeldwicklung mit der Polpaarzahl p zum Erzeugen eines Antriebsdrehfeldes und zum gesteuerten Drehen des antreibenden Rotors um seine Rotationsachse, sowie eine Steuerwicklung mit der Polpaarzahl p+1 oder p-1 zum Erzeugen eines dem Antriebsdrehfeld überlagerten Steuerfeldes, um die radiale Lage (zwei Freiheitsgrade) des angetriebenen Rotors in seiner Rotationsebene geregelt, d.h. aktiv zu stabilisieren.

Bei der Rotationsmaschine kann der antreibende Teil des Integralrotors, scheibenförmig, ringförmig oder glockenförmig gestaltet sein und der Rotor kann axial, sowie gegen Verkippung gegenüber der Statorebene passiv durch Reluktanzkräfte stabilisiert sein.

Die Rotationsmaschine kann eine Rotorgeometrie haben, die so ist, dass mindestens einer der nicht aktiv stabilisierten Freiheitsgrade des Rotors, passiv durch hydrostatische oder hydrodynamische Kräfte, durch aerostatische, aerodynamische Kräfte oder durch Gravitationskräfte stabilisiert ist.

Bei einer derartigen Rotationsmaschine kann der Stator zusammen mit dem antreibenden Rotor einen permanentmagnetisch erregten Synchronmotor oder Reluktanzmotor bilden oder der Stator kann zusammen mit dem antreibenden Rotor einen Aussenläufermotor bilden.

Der permanentmagnetisch antreibende Rotor kann als Ringmagnet, Scheibenmagnet oder als Schalenmagnet ausgebildet sein und es kann auch das Rotorrückschlusseisen oder das Eisenkreuz Teil des Integralrotors sein.

In einer derartigen Rotationsmaschine kann der Integralrotor zusammen mit einem Teil der Arbeitsvorrichtung als Einheit ausgebildet sein, welche vom Antriebsstator abhebbar ist.

Der Antriebsstator der Rotationsmaschine kann aus einzelnen, stabförmigen, radial um den Rotor angeordneten Spulen mit einem gemeinsamen magnetischen Rückschluss gebildet sein und jede der Spulen kann Teilwicklungen für jeden Wicklungsstrang der Antriebswicklung und der Steuerwicklung aufweisen.

Bei der Rotationsmaschine kann die Windungszahl der Teilwicklungen so gewählt sein, dass die Antriebsdurchflutung und die Steuerdurchflutung geometrisch wenigstens angenähert sinusförmig verteilt sind. Für eine zweiphasige Lager-Antriebseinheit kann zum Beispiel die Windungszahl jeder Teilwicklung der ersten Phase (sowohl der Antriebswicklung, als auch der Steuerwicklung) einer gegebenen Windungszahl N₁ mal dem Kosinus des elektrischen Winkels α ihrer Lage, also N₁ cos(pα) für die Antriebswicklung und N₁ cos{(p±1)α} für die Steuerwicklung sein und die Windungszahl jeder Teilwicklung der zweiten Phase (wiederum sowohl der Antriebswicklung als auch der Steuerwicklung) einer gegebenen Windungszahl N₂ mal den Sinus des elektrischen Winkels α ihrer Lage, also N₂ sind(pα) für die Antriebswicklung und N₂ sin{(p±1)α} für die Steuerwicklung sein, oder umgekehrt.

Der Antriebsstator der Rotationsmaschine kann bezogen auf den Rotor in axialer Richtung, aus tempelsäulenartig angeordneten Spulen, als Tempelmotor mit einem gemeinsamen magnetischen Rückschluss ausgebildet sein, wobei der Fluss an einem Spulenende, auf der dem magnetischen Rückschluss gegenüberliegenden Seite, durch eine zum Rotor hin zeigende L-förmige Verlängerung des Spulenkerns radial zum Rotor hin geführt wird und die Spulen Teilwicklungen für jeden Wicklungsstrang der Antriebswicklung und der Steuerwicklung aufweisen, wobei einzelne Teilwicklungen die Windungszahl null haben können und die wenigstens angenähert sinusförmige geometrische Verteilung der Antriebsdurchflutung und der Steuerdurchflutung durch das Windungszahlverhältnis zwischen den einzelnen Teilspulen gebildet wird.

Bei der Rotationsmaschine können im Luftspalt zwischen Stator und Rotor und/oder in den Spulenkernen Flusssonden angebracht werden, um eine oder mehrere Teilflusskomponenten zu bestimmen, die mit Mitteln zum Übermitteln der gemessenen Teilflusskomponenten an die Antriebs- und Positionsregler des lagerlosen Motors übertragen werden.

Bei der Rotationsmaschine kann durch gewichtete Summation der Teilflüsse über jeweils den halben Umfang in x-Richtung und y-Richtung der Rotationsebene des antreibenden Rotors und in der Gegenrichtung, Betragsbildung und anschliessende Differenzbildung des Anteils in x-Richtung und in Gegenrichtung sowie des Anteils in y-Richtung und in Gegenrichtung, die Rotorposition bestimmt werden. Aus den Teilflüssen kann der Winkel und/oder der Betrag des Antriebsflusswinkels bestimmt werden.

Bei der Rotationsmaschine können in einzelnen Nuten oder zwischen einzelnen Spulenkernen des Antriebsstators Wirbelstromdistanzsensoren angeordnet sein, die den Abstand zu einer leitfähigen Schicht im antreibenden Rotor, in der Rotorebene (xy-Ebene) messen.

Solche Wirbelstromdistanzsensoren können aus einem Metallring, oder einer dünnen Metallschicht oder aus dem leitfähigen Magnetmaterial des antreibenden Rotormagneten bestehen.

In X- und Y-Richtung können jeweils zwei gegenüberliegende Distanzsensoren eingesetzt sein und die Komponenten der Position des antreibenden Rotors in der Rotorebene (X-Y-Ebene) kann aus der Differenz der Sensorsignale gegenüberliegender Sensoren bestimmt werden. Der Antriebsstator der Rotationsmaschine kann zusätzlich zur Antriebs- und Steuerwicklung Sensorwicklungen aufweisen.

Die axiale Position des antreibenden und des angetriebenen Rotors der Rotationsmaschine, d.h. des Integralrotors, kann mit einem Magnetisierungsstrom in der Antriebswicklung oder mit einer zusätzlichen Axialwicklung auf der einen Seite des Rotors geregelt und stabilisiert sein; dies ist jedoch nicht Gegenstand der Erfindung.

Bei einer Rotationsmaschine kann zwischen dem Antriebsstator und Integralrotor der Rotationsmaschine ein Spaltrohr angeordnet sein.

Die mit der Rotationsmaschine angetriebene Arbeitsvorrichtung kann beispielsweise eine Rotationspumpe, insbesondere eine medizinische Pumpe zum Pumpen von Blut, ein Rührwerk, eine Turbomaschine, eine Spindel, eine Zentrifuge oder eine Galette sein. Die Rotationspumpe kann beispielsweise eine Axialpumpe, eine Zentrifugalpumpe, eine Seitenkanalpumpe, eine Peripheralpumpe oder eine Teslapumpe sein. Die als Rotationspumpe ausgebildete Rotationsmaschine kann ein geschlossenes Pumpengehäuse aufweisen, das den Integralrotor enthält, welcher den antreibenden Rotor des Motors und den angetriebenen Rotor der Pumpe umfasst. Das Pumpengehäuse kann mit Vorteil auf mindestens einer Seite frei zugänglich und austauschbar am Antriebsstator angebracht sein.

Eine solche als Axialpumpe ausgebildete Rotationsmaschine kann einen ringförmigen antreibenden Rotor aufweisen, der - als Hohlwellenläufer ausgebildet - das Flügelrad der Axialpumpe ringförmig umgibt.

Bei einer solchen als Axialpumpe ausgebildeten Rotationsmaschine kann das Pumpengehäuse auf einer Seite einen Einlassstutzen und auf einer anderen Seite einen Auslassstutzen aufweisen. Über diese Stutzen kann die Pumpe in den Förderkreislauf eingefügt werden und zwar mit Vorteil derart, dass das Pumpengehäuse mit dem Pumpenrad nach der Demontage der vorzugsweise schlauchartigen Verbindungen vom antreibenden Stator entfernbar ist.

Die Rotationsmaschine kann auch als Zentrifugalpumpe ausgebildet sein, deren ringförmiger oder scheibenförmigen antreibender Rotor in ein Flügelrad, vorzugsweise aus Kunststoff integriert oder an das Flügelrad angebaut ist.

Bei einer als Rotationspumpe ausgebildeten Rotationsmaschine, kann das axiale Rotorpositionssignal oder das Regelsignal der Regelanlage, das zur axialen Stabilisierung des Rotors genutzt wird auch zum Bestimmen des Pumpendrucks genutzt werden.

Bei einer als Rotationspumpe ausgebildeten Rotationsmaschine, können Mittel zum Bestimmen des Durchflusses von Fluid durch die Pumpe aus dem axialen Rotorpositionssignal, der Drehzahl des Rotors sowie der drehmomentbildenden Komponente des Antriebsstroms (Q-Komponente) vorgesehen sein.

Bei einer als Zentrifugalpumpe ausgebildeten Rotationsmaschine, kann das Pumpengehäuse auf beiden axialen Seiten einen Einlass und zwei Auslässe aufweisen.

Bei einer Rotationsmaschine nach der Erfindung kann die axiale Länge des antreibenden Rotors mit Vorteil kleiner oder gleich dem halben Durchmesser dieses Rotors sein.

Aus den vorgenannten generellen Ausführungen hinsichtlich einer Rotationsmaschine ist klar, dass sich die Erfindung auch auf eine Rotationspumpe für Fluide bezieht, mit einem hermetisch abgeschlossenen Pumpengehäuse mit mindestens einem Einlass und mindestens einem Auslass für das Fluid und mit einem Pumpenrotor, der durch eine magnetische Lagervorrichtung gelagert und durch eine berührungsfreie elektrische Antriebsvorrichtung antreibbar ist, wobei die magnetische Lagervorrichtung und die berührungsfreie elektrische Antriebsvorrichtung einen lagerlosen Drehfeldmotor mit einem gemeinsamen Lager/Antriebs-Rotor bilden, der mit dem Pumpenrotor zusammen als Integralrotor 14 ausgeführt ist, wobei der lagerlose Drehfeldmotor eine Antriebswicklung mit einer Polpaarzahl p und eine Steuerwicklung mit einer Polpaarzahl p+1 oder p-1 besitzt, derart, dass die Rotation des Integralrotors 14 um seine Rotationsachse mittels der Antriebswicklung und die Position des Integralrotors 14 in der senkrecht zur Rotationsachse verlaufenden Ebene (X-Y-Ebene) mittels der Steuerwicklung aktiv steuerbar sind, und die Position des Integralrotors 14 längs der Rotationsachse und seine Verkippung aus der genannten Ebene (X-Y-Ebene) durch Reluktanzkräfte passiv stabilisiert sind. Die Rotationspumpe kann dabei ein Pumpengehäuse 8 aufweisen bei welchem der Integralrotor 14, der darin aufgenommenen ist, von aussen frei zugänglich und ausbaubar angeordnet ist.

Der Integralrotor 14 der Rotationspumpe kann eine ringartige Rotorscheibe 16 aufweisen, in welcher die elektromagnetisch wirksamen Bauteile 26, 28, 30 enthalten sind, und an welcher die Rotorschaufeln 18, 19 befestigt sind. Der Pumpenrotor und die elektromagnetisch wirksamen Bauteile 26, 28, 30 können auch in miteinander verschweisste Teile der Rotorscheibe 16 eingebettet oder an ihr angespritzt sein. Bei der Rotationspumpe können die vom geförderten Fluid berührten Flächen des Pumpengehäuses 8 und des Integralrotors 14 aus Kunststoff bestehen.

Das Pumpengehäuse 8 kann einen weiteren axialen Einlass 11 aufweisen, der dem ersten axialen Einlass 10 gegenüberliegt. Das Pumpengehäuse 8 kann auch einen weiteren, mindestens annähernd radialen, Auslass 13 besitzen, der zentralsymmetrisch zum ersten radialen Auslass 12 angeordnet ist (Fig. 3). Die Rotationspumpe kann mit Vorteil als Axialpumpe ausgebildet sein (Fig. 9).

Die Rotorschaufeln 18 können als Flügel eines Flügelrades 24 ausgebildet sein, das in der mittigen Ausnehmung der ringartigen Rotorscheibe 16 befestigt ist.

Die Rotationspumpe kann auch eine Zentrifugalpumpe sein (Fig. 10 bzw. Fig. 11). Bei der Rotationspumpe können die Rotorschaufeln 18 an einer Fläche der Rotorscheibe 16 angeordnet sein. Die Rotorschaufeln 18, 19 können an beiden Flächen der Rotorscheibe 16 angeordnet sein.

Der Lager/Antriebs-Stator des Drehfeldmotors der Rotationspumpe kann mehrere längliche, um den Integralrotor 14 angeordnete Spulenkerne 34 mit einem gemeinsamen magnetischen Rückschluss aufweisen, wobei jeder Spulenkern 34 eine Teilwicklung des Wicklungsstranqs der Antriebswicklung mit der Polpaarzahl p und eine Teilwicklung des Wicklungsstrangs der Steuerwicklung mit der Polpaarzahl p+1 oder p-1 enthält, wobei eine sinusförmige geometrische Verteilung der Antriebsdurchflutung und der Steuerdurchflutung durch das Verhältnis der Windungszahlen der Teilwicklungen eines Wicklungsstranges angenähert ist. Dabei kann eine der Teilwicklungen die Windungszahl null aufweisen.

Die Spulenkerne 34 der Rotationspumpe können radial zur Rotationssachse 3 des Integralrotors 14 angeordnet sein und die Spulenkerne 34 und das Rückschlusseisen können eine Einheit bilden wobei diese Einheit aus einzelnen Blechen mit langen Nuten geschichtet ist.
Andere Spulenkerne 35 können die Form eines "L" aufweisen, deren einer Schenkel 35a jeweils parallel zur Rotationsachse 3 des Integralrotors 14 angeordnet ist und deren anderer Schenkel 35b jeweils radial einwärts zur Rotationsachse 3 des Integralrotors 14 gerichtet ist, um den Fluss radial zum Integralrotor 14 zu führen.

Der Drehfeldmotor kann auch ein Synchronmotor sein, wobei dieser ein Reluktanzmotor oder ein permanentmagnetisch erregbarer Synchronmotor ist.

In der Rotationspumpe kann eine Einrichtung vorgesehen sein, um mindestens eine Teilflusskomponente zu ermitteln, durch welche der für die Antriebsregelung des lagerlosen Motors notwendige Antriebsflusswinkel bestimmbar ist. Diese Einrichtung kann mindestens eine Flussonde 50x, 50y, 51x, 51y, 50a bis 50h aufweisen. Weiter kann die Rotationspumpe eine Detektoreinrichtung zur Bestimmung der Position des Integralrotors 14 aufweisen. Diese Detektoreinrichtung kann einen X-Y-Detektor aufweisen, um die Position des Integralrotors 14 längs der Achsen X und Y zu bestimmen. Der X-Y-Detektor kann mindestens eine Flussonde 50x, 50y, 51x, 51y, 50a bis 50h aufweisen.

Es können mehrere symmetrisch verteilte Flussonden 50x, 50y, 51x, 51y, 50a bis 50h vorgesehen sein, zur Messung von Teilflüssen an diskreten Stellen, und aus den Teilflüssen kann zusätzlich zum Drehwinkel des Motors über den Antriebsflusswinkel, durch gewichtete Summation der Teilflüsse über jeweils den halben Umfang in positiver und negativer X- und Y-Richtung, Betragsbildung und anschliessende Differenzbildung des Anteils in positiver und negativer X-Richtung sowie des Anteils in positiver und negativer Y-Richtung die X- und die Y-Komponente der Position des Integralrotors 14 bestimmt werden.

Bei einer derartigen Rotationspumpe kann mindestens eine Flussonde 50x, 50y, 51x, 51y, 50a bis 50h im Luftspalt zwischen dem Pumpengehäuse 8 und dem Lager/Antriebsstator angeordnet sein, wobei die mindestens eine Flussonde 50x, 50y, 51x, 51y, 50a bis 50h im Spulenkern 34, 35 angeordnet sein kann. Die mindestens eine Flussonde kann ein Hallelement enthalten. Die mindestens eine Flussonde könnte aber auch eine magnetoresistive Flussonde sein.

Die mindestens eine Flussonde kann auf einem Zahn des Stators des Drehfeldmotors befestigt, beispielsweise aufgeklebt sein. Die mindestens eine Flussonde kann in einer Ausnehmung zwischen zwei Zähnen des Stators des Drehfeldmotors eingebettet sein. Der X-Y-Detektor kann mindestens einen Wirbelstromdistanzsensor enthalten, um damit den Abstand zu einer leitfähigen Schicht im Integralrotor in der X-Y-Ebene zu messen.

Die leitfähige Schicht 30 kann aus einem Metallring oder einer dünnen Metallschicht oder aus dem, aus leitfähigem Material, beispielsweise NdFe, gefertigten Magneten 28 bestehen.

Bei der Rotationspumpe können in X- und in Y-Richtung jeweils zwei einander gegenüberliegende Sensorsignale ermittelnde Wirbelstromdistanzsensoren vorgesehen sein, um die Komponenten der Position des Integralrotors in der X-Y-Rotorebene aus der Differenz der Sensorsignale der einander gegenüberliegenden Wirbelstromdistanzsensoren zu gewinnen.

Der X-Y-Detektor kann Sensorwicklungen enthalten, welche zusätzlich zur Antriebs- und Steuerwicklung im Stator des Drehfeldmotors angeordnet sind, um die Position des Integralrotors 14 zu ermitteln, indem die elektrischen Impedanz der Sensorwicklungen bestimmt wird. Der X-Y-Detektor kann auch eine optische Einrichtung enthalten, um die Position des Integralrotors mit Licht, dessen Wellenlänge im optischen Bereich des Fluids liegt, zu messen. Die Detektoreinrichtung kann auch einen Z-Detektor zur Bestimmung der axialen Position des Integralrotors und zum Ermitteln eines entsprechenden Z-Positionssignals umfassen.

Das Z-Positionssignal kann den Ist-Wert für die Ermittlung eines Regelsignals für eine regeltechnische Stabilisierung der axialen Position des Integralrotors bilden. Zur regeltechnischen Stabilisierung der axialen Position des Integralrotors kann ein Magnetisierungsstrom in der Antriebswicklung mit einer Stromkomponente in der Flussrichtung aufgebracht werden.

Es kann auch eine Druckmessvorrichtung angeordnet sein, um aus dem Z-Positionssignal oder aus dem daraus ermittelten Regelsignal den Pumpendruck zu bestimmen.

Es kann zudem eine Durchflussbestimmungsvorrichtung vorgesehen sein, um aus dem Z-Positionssignal und der Drehzahl des Integralrotors und der drehmomentbildenden Komponente des Antriebsstroms den Durchfluss zu bestimmen.

Eine Rotationsmaschine bzw. Rotationspumpe nach der Erfindung kann auch miniaturisiert, beispielsweise als in Tier oder Mensch implantierbare Blutpumpe ausgeführt sein.

## Patentansprüche

1. Rotationsmaschine mit einem angetriebenen Rotor (2) und mit einem elektrischen Motor (4,14), der einen Stator (4) und einen antreibenden Rotor (14) umfasst, wobei der Stator (4) auch als elektromagnetisches Lager für den antreibenden Rotor (14) ausgebildet ist und der antreibende Rotor (14) des Elektromotors (4, 14) mit dem angetriebenen Rotor der Rotationsmaschine eine Rotoreinheit, d.h. einen Integralrotor (14) bildet, dadurch gekennzeichnet, dass insgesamt nur ein einziger Stator (4) vorgesehen ist, wobei dieser einzige Stator (4) den Integralrotor (14) sowohl lagert als auch antreibt, und wobei die axiale Länge des antreibenden Rotors kleiner oder gleich dem halben Durchmesser dieses Rotors ist und des Integralrotor axial sowie gegen Verkippung gegenüber der Statorebene passiv durch Reluktanzkräfte stabilisiert ist.

2. Rotationsmaschine nach Anspruch 1, mit
einer Antriebs-/Drehfeldwicklung mit der Polpaarzahl p zum Erzeugen eines Antriebsdrehfeldes und zum gesteuerten Drehen des antreibenden Rotors um seine Rotationsachse, und mit
einer Steuerwicklung mit der Polpaarzahl p+1 oder p-1 zum Erzeugen eines dem Antriebsdrehfeld überlagerten Steuerfeldes,
um die radiale Lage, also zwei Freiheitsgrade, des angetriebenen Rotors in seiner Rotationsebene geregelt, d.h. aktiv, zu stabilisieren.

3. Rotationsmaschine nach Anspruch 1 oder 2, bei welcher der antreibende Teil des Integralrotors (14) scheibenförmig, ringförmig, oder glockenförmig gestaltet ist.

4. Rotationsmaschine nach einem der Ansprüche 1 bis 3, bei welcher der Stator zusammen mit dem antreibenden Rotor einen Aussenläufermotor bildet.

5. Rotationsmaschine nach einem der Ansprüche 1 bis 4, bei welcher der Integralrotor (14) zusammen mit einem Teil (8) einer Arbeitsvorrichtung als Einheit ausgebildet ist, welche vom Antriebsstator (4) abhebbar ist.

6. Rotationsmaschine nach einem der Ansprüche 1 bis 5, bei welcher der Antriebsstator (4) aus einzelnen, stabförmigen, radial um den Rotor (14) angeordneten Spulen (36) mit einem gemeinsamen magnetischen Rückschluss (38) aufgebaut ist und jede Spule Teilwicklungen für jeden Wicklungsstrang der Antriebswicklung und der Steuerwicklung aufweisen.

7. Rotationsmaschine nach Anspruch 6, bei welcher die Windungszahl der Teilwicklungen so gewählt ist, dass die Antriebsdurchflutung und die Steuerdurchflutung geometrisch wenigstens angenähert sinusförmig ist.

8. Rotationsmaschine nach Anspruch 7, mit zweiphasiger Wicklung, bei welcher die Windungszahl jeder Teilwicklung der ersten Phase einer gegebenen Windungszahl N₁ mal dem Kosinus des elektrischen Winkels α ihrer Lage, also N₁ x cos (p α) für die Antriebswicklung und N₁ x cos{(p±1)α} für die Steuerwicklung ist, und bei welcher die Windungszahl jeder Teilwicklung der zweiten Phase einer gegebenen Windungszahl N₂ mal dem Sinus des elektrischen Winkels α ihrer Lage, also N₂ x sin (p α) für die Antriebswicklung und N₂ x sin{(p±1)α} für die Steuerwicklung ist, oder umgekehrt.

9. Rotationsmaschine nach einem der Ansprüche 1 bis 8, bei welchem der Antriebsstator bezogen auf den Rotor in axialer Richtung, aus tempelsäulenartig angeordneten Spulen, als Tempelmotor mit einem gemeinsamen magnetischen Rückschluss ausgebildet ist wobei der Fluss an einem Spulenende, auf der dem magnetischen Rückschluss gegenüberliegenden Seite, durch eine zum Rotor hin zeigende L-förmige Verlängerung des Spulenkerns (35) radial zum Rotor hin geführt wird und die Spulen Teilwicklungen für jeden Wicklungsstrang der Antriebswicklung und der Steuerwicklung aufweisen, wobei einzelne Teilwicklungen die Windungszahl null haben können und die wenigstens angenähert sinusförmige geometrische Verteilung der Antriebsdurchflutung und der Steuerdurchflutung durch das Windungszahlverhältnis zwischen den einzelnen Teilspulen gebildet wird.

10. Rotationsmaschine nach einem der Ansprüche 1 bis 9, bei welcher in einzelnen Nuten oder zwischen einzelnen Spulenkernen des Antriebsstators Wirbelstromdistanzsensoren angeordnet sind, die den Abstand zu einer leitfähigen Schicht (30) im antreibenden Rotor in der Rotorebene messen.

11. Rotationsmaschine nach einem der Ansprüche 1 bis 10, bei welcher in X- und Y-Richtung jeweils zwei gegenüberliegende Distanzsensoren eingesetzt sind und die Komponenten der Position des antreibenden Rotors in der Rotorebene aus der Differenz der Sensorsignale gegenüberliegender Sensoren bestimmt wird.

12. Rotationsmaschine nach Anspruch 1 bis 9, bei welcher im Antriebsstator zusätzlich zur Antriebs- und Steuerwicklung Sensorwicklungen (60x,60y,61x,61y) angeordnet sind.

13. Rotationsmaschine nach einem der Ansprüche 1 bis 12, bei der zwischen Antriebsstator und Integralrotor ein Spaltrohr (32) angeordnet ist.

14. Rotationsmaschine nach einem der Ansprüche 1 bis 13, als Rotationspumpe ausgebildet, mit einem geschlossenen Pumpengehäuse (8), das den Integralrotor (14) enthält, welcher den antreibenden Rotor des Motors und den angetriebenen Rotor der Pumpe umfasst.

15. Rotationsmaschine nach einem der Ansprüche 1 bis 14, als Rotationspumpe ausgebildet, mit einem Pumpengehäuse (8), das auf mindestens einer Seite frei zugänglich und austauschbar am Antriebsstator angebracht ist.

16. Rotationsmaschine nach einem der Ansprüche 1 bis 15, als Axialpumpe ausgebildet, mit einem ringförmigen antreibenden Rotor, der - als Hohlwellenläufer ausgebildet - das Flügelrad der Axialpumpe ringförmig umgibt.

17. Rotationsmaschine nach einem der Ansprüche 1 bis 16, als Axialpumpe ausgebildet, deren Pumpengehäuse auf einer Seite einen Einlassstutzen (10) und auf einer anderen Seite einen Auslassstutzen (12) aufweist, über welche Stutzen die Pumpe in den Förderkreislauf eingefügt wird, derart, dass das Pumpengehäuse (8) mit Pumpenrad nach Demontage der vorzugsweise schlauchartigen Verbindungen vom antreibenden Stator entfernbar ist.

18. Rotationsmaschine nach einem der Ansprüche 1 bis 15, als Zentrifugalpumpe ausgebildet, deren ringförmiger oder scheibenförmiger antreibender Rotor in ein Flügelrad, vorzugsweise aus Kunststoff, integriert ist.

19. Rotationsmaschine nach einem der Ansprüche 1 bis 18, als Rotationspumpe ausgebildet, bei welcher das axiale Rotorpositionssignal oder das Regelsignal einer Regelanlage, das zur axialen Stabilisierung des Rotors notwendig ist, zum Bestimmen des Pumpendrucks genutzt wird.

20. Rotationsmaschine nach einem der Ansprüche 1 bis 18, als Rotationspumpe ausgebildet, mit Mitteln zum Bestimmen des Durchflusses von Fluid durch die Pumpe aus dem axialen Rotorpositionssignal, der Drehzahl des Rotors sowie dem Antriebsstrom.

21. Rotationsmaschine nach einem der Ansprüche 1 bis 20, dadurch gekennzeichnet, dass im Stator Mittel vorhanden sind, welche im Elektromotor einen Unipolarfluss (52,53,54,55) erzeugen und dass der Stator eine zweipolige Drehfeldwicklung enthält, über welche sich auf den Rotor wirkende, radiale magnetische Kräfte steuern lassen und dass der Stator eine weitere Drehfeldwicklung mit einer Polpaarzahl p>=2 enthält, über welche sich ein Drehfeld zum Antreiben des Rotors erzeugen lässt.

22. Rotationsmaschine nach Anspruch 21, dadurch gekennzeichnet, dass der Unipolarfluss im Elektromotor durch Permanentmagnete (50,51) erzeugt wird.

## Claims

1. Rotary machine comprising a driven rotor (2) and an electric motor (4, 14) having a stator (4) and a driving rotor (14), with the stator (4) also being executed as an electromagnetic bearing for the driving rotor (14) and the driving rotor (14) of the electric motor (4, 14) together with the driven rotor of the rotary machine forming a rotor unit, i.e. an integral rotor (14), characterised in that in total only one single stator (4) is provided, said single stator (4) both supporting and driving the integral rotor (14) and wherein the axial length of the driving rotor is smaller than or equal to half the diameter of this rotor and the integral rotor is passively stabilised by reluctance forces against tilting with respect to the stator plane.

2. Rotary machine in accordance with claim 1 comprising
a driving field winding/rotary field winding with the number of pole pairs p for the production of a rotary driving field and for the controlled rotation of the driving rotor about its axis of rotation, and
a control winding with the number of pole pairs p+1 or p-1 for the production of a control field superimposed on the rotary driving field,
in order to regulate the radial position of the driven rotor in its plane of rotation, that is two degrees of freedom, in a controlled manner, i.e. to actively stabilise it.

3. Rotary machine in accordance with claim 1 or claim 2 in which the driving part of the integral rotor (14) is designed to be disc-shaped, ring-shaped or bell-shaped.

4. Rotary machine in accordance with one of the claims 1 to 3 in which the stator together with the driving rotor forms an external rotor motor.

5. Rotary machine in accordance with one of the claims 1 to 4 in which the integral rotor (14) is constructed together with a part (8) of the working apparatus as a unit which can be lifted off from the drive stator (4).

6. Rotary machine in accordance with one of the claims 1 to 5 in which the drive stator (4) is built up of individual, rod-shaped coils (36) arranged radially about the rotor (14) with a common magnetic yoke (38) and each coil has partial windings for each winding string of the drive winding and of the control winding.

7. Rotary machine in accordance with claim 6 in which the number of turns of the partial windings is chosen in such a manner that the drive flux and the control flux are geometrically at least approximately sinusoidal.

8. Rotary machine in accordance with claim 7 with a two phase winding in which the number of turns of each partial winding of the first phase is a given number of turns N₁ times the cosine of the electric angle α of its position, that is N₁ x cos (p α) for the drive winding and N₁ x cos {(p±1)α} for the control winding, and in which the number of turns of each partial winding of the second phase is a given number of turns N₂ times the sine of the electric angle α of its position, that is N₂ x sin (p α) for the drive winding and N₂ x sin {(p±1)α} for the control winding or vice versa.

9. Rotary machine in accordance with one of the claims 1 to 8 in which the drive stator is executed, with respect to the rotor in the axial direction, of coils arranged in the manner of pillars of a temple as a temple motor having a common magnetic yoke, with the flux at one coil end, at the side lying opposite the magnetic yoke, being conducted radially to the rotor through an L-shaped extension of the coil core (35) pointing towards the rotor, and with the coils having partial windings for each winding string of the drive winding and the control winding, where individual partial windings can have zero as the number of turns and the at least approximately sinusoidal geometrical distribution of the drive flux and the control flux is formed by the winding turn ratio between the individual partial coils.

10. Rotary machine in accordance with one of the claims 1 to 9 in which eddy current distance sensors are arranged in individual grooves or between individual coil cores of the drive stator and measure the distance to a conducting layer (30) in the driving rotor in the rotor plane.

11. Rotary machine in accordance with one of the claims 1 to 10 in which two oppositely disposed distance sensors are used in the X direction and the Y direction in each case and the components of the position of the driving rotor in the rotor plane are determined from the difference of the sensor signals of oppositely disposed sensors.

12. Rotary machine in accordance with one of the claims 1 to 9 in which sensor windings (60x, 60y, 61x, 61y) are arranged in the drive stator in addition to the drive and control windings.

13. Rotary machine in accordance with one of the claims 1 to 12 in which a split tube (32) is arranged between the drive stator and the integral rotor.

14. Rotary machine in accordance with one of the claims 1 to 13, executed as a rotary pump, with a closed pump housing (8) which contains the integral rotor (14) which encloses the driving rotor of the motor and the driven rotor of the pump.

15. Rotary machine in accordance with one of the claims 1 to 14, executed as a rotary pump, with a pump housing (8) that is arranged on the drive stator in such a manner as to be freely accessible and replaceable from at least one side.

16. Rotary machine in accordance with one of the claims 1 to 15, executed as an axial pump, with a ring-shaped driving rotor which - executed as a hollow shaft rotor - surrounds the vaned wheel of the axial pump in a ring-like manner.

17. Rotary machine in accordance with one of the claims 1 to 16, executed as an axial pump, the pump housing of which has an inlet stub (10) at the one side and an outlet stub (12) at the other side via which stubs the pump is inserted into the forwarding circuit in such a manner that the pump housing (8) can be removed with the pump wheel from the driving stator after removal of the preferably hose-like connections.

18. Rotary machine in accordance with one of the claims 1 to 15, executed as a centrifugal pump, whose ring-shaped or disc-shaped driving rotor is integrated into a vaned wheel, preferably of plastic.

19. Rotary machine in accordance with one of the claims 1 to 18, executed as a rotary pump, in which the axial rotor position signal or the control signal of the control system which is required for the axial stabilisation of the rotor is used for the determination of the pump pressure.

20. Rotary machine, in accordance with one of the claims 1 to 18, executed as a rotary pump, with means for determining the through-flow of fluid through the pump from the axial rotor position signal, the speed of rotation of the rotor and from the driving current.

21. Rotary machine in accordance with one of the claims 1 to 20 characterised in that means are present in the stator which produce a unipolar flux (52, 53, 54, 55) in the electric motor; and in that the stator has a bipolar rotary field winding via which radial magnetic fields acting on the rotor can be controlled; and in that the stator has a further rotary field winding with a number of pole pairs p>=2 via which a rotary field can be produced for driving the rotor.

22. A rotary machine in accordance with claim 21 characterised in that the unipolar flux in the electric motor is produced by permanent magnets (50, 51).

## Revendications

1. Machine rotative avec un rotor entraîné (2) et avec un moteur électrique (4,14), qui comporte un stator (4) et un rotor d'entraînement (14), où le stator (4) est également réalisé comme palier électromagnétique pour le rotor d'entraînement (14) et où le rotor d'entraînement (14) du moteur électrique (4,14) forme avec le rotor entraîné de la machine rotative une unité rotorique, c'est-à-dire un rotor intégral (14), caractérisée en ce qu'au total, seulement un stator unique (4) est prévu, où ce stator unique (4) à la fois loge le rotor intégral (14) et entraîne celui-ci, et où la longueur axiale du rotor d'entraînement est plus petite ou égale au demi-diamètre de ce rotor, et le rotor intégral est stabilisé axialement et à l'encontre d'un basculement par rapport au plan de stator passivement par des forces de réluctance.

2. Machine rotative selon la revendication 1, avec un enroulement d'entraînement/de champ magnétique tournant avec le nombre de paires de pôles p pour produire un champ rotatif d'entraînement et pour la rotation commandée du rotor d'entraînement autour de son axe de rotation, et avec un enroulement de commande avec le nombre de paires de pôles p+1 ou p-1 pour produire un champ de commande superposé au champ tournant d'entraînement, pour stabiliser d'une manière réglée, c'est-à-dire activement, la position radiale, donc deux degrés de liberté, du rotor entraîné dans son plan de rotation.

3. Machine rotative selon la revendication 1 ou 2, où la partie d'entraînement du rotor intégral (14) est réalisée en forme de disque, en forme d'anneau ou en forme de cloche.

4. Machine rotative selon l'une des revendications 1 à 3, où le stator ensemble avec le rotor d'entraînement forme un moteur à rotor externe.

5. Machine rotative selon l'une des revendications 1 à 4, où le rotor intégral (14) ensemble avec une partie (8) d'un dispositif de travail est réalisé comme unité apte à être retirée du stator d'entraînement (4).

6. Machine rotative selon l'une des revendications 1 à 5, où le stator d'entraînement (4) est constitué de bobines individuelles (36), en forme de tige, disposées radialement autour du rotor (14), avec une fermeture magnétique commune (38), et où chaque bobine présente des enroulements partiels pour chaque faisceau de l'enroulement d'entraînement et de l'enroulement de commande.

7. Machine rotative selon la revendication 6, où le nombre de spires des enroulements partiels est choisi de façon que le flux d'entraînement et le flux de commande soient géométriquement au moins approximativement sinusoïdaux.

8. Machine rotative selon la revendication 7, avec un enroulement à deux phases, où le nombre de spires de chaque enroulement partiel de la première phase d'un nombre de spires donné N₁ fois le cosinus de l'angle électrique a de leur position, est donc N₁ x cos (p α) pour l'enroulement d'entraînement et N₁ x cos {(p±1)α} pour l'enroulement de commande, et où le nombre de spires de chaque enroulement partiel de la deuxième phase d'un nombre de spires donné N₂ fois le sinus de l'angle électrique a de leur position, est donc N₂ x sin (p α) pour l'enroulement d'entraînement et N₂ x sin{(p±1)α} pour l'enroulement de commande, ou inversement.

9. Machine rotative selon l'une des revendications 1 à 8, où le stator d'entraînement, par rapport au rotor dans la direction axiale, est réalisé à partir de bobines disposées à la manière de colonnes de temple, comme moteur temple avec une fermeture magnétique commune, où le flux est guidé à une extrémité de bobine, sur le côté opposé à la fermeture magnétique, par un prolongement en forme de L du noyau de bobine (35) orienté vers le rotor, radialement au rotor, et les bobines présentent des enroulements partiels pour chaque faisceau de l'enroulement d'entraînement et de l'enroulement de commande, où les enroulements partiels individuels peuvent avoir le nombre de spires zéro, et la répartition géométrique au moins approximativement sinusoïdale du flux d'entraînement et du flux de commande est réalisée par le rapport du nombre de spires entre les bobines partielles individuelles.

10. Machine rotative selon l'une des revendications 1 à 9, où sont disposés dans des rainures individuelles ou entre des noyaux de bobine individuels du stator d'entraînement des capteurs d'espacement de courant tourbillonnaire qui mesurent l'espacement à une couche conductrice (30) dans le rotor d'entraînement dans le plan de rotor.

11. Machine rotative selon l'une des revendications 1 à 10, dans laquelle sont mis en place dans la direction X et Y respectivement deux capteurs d'espacement opposés, et les composantes de la position du rotor d'entraînement dans le plan de rotor sont déterminées à partir de la différence des signaux de capteur opposés.

12. Machine rotative selon la revendication 1 à 9, dans laquelle sont disposés dans le stator d'entraînement, en plus de l'enroulement d'entraînement et de commande, des enroulements de capteur (60x, 60y, 61x, 61y).

13. Machine rotative selon l'une des revendications 1 à 12, dans laquelle est disposé entre le stator d'entraînement et le rotor intégral une gaine (32).

14. Machine rotative selon l'une des revendications 1 à 13, réalisée comme pompe rotative, avec un corps de pompe fermé (8) qui comporte le rotor intégral (14) qui comprend le rotor d'entraînement du moteur et le rotor entraîné de la pompe.

15. Machine rotative selon l'une des revendications 1 à 14, réalisée comme pompe rotative, avec un corps de pompe (8) qui est disposé d'une manière librement accessible sur au moins un côté et de manière échangeable au stator d'entraînement.

16. Machine rotative selon l'une des revendications 1 à 15, réalisée comme pompe axiale, avec un rotor d'entraînement annulaire qui - réalisé comme rotor à arbre creux - entoure en forme d'anneau la roue à ailettes de la pompe axiale.

17. Machine rotative selon l'une des revendications 1 à 16, réalisée comme pompe axiale, dont le corps de pompe présente sur un côté une tubulure d'entrée (10) et sur l'autre côté une tubulure de sortie (12), par lesquelles la pompe est insérée dans le circuit de convoyage de telle sorte que le corps de pompe (8) avec la roue de pompe, après le démontage des liaisons de préférence tubulaires, peut être retiré du stator d'entraînement.

18. Machine rotative selon l'une des revendications 1 à 15, réalisée comme pompe centrifuge, dont le rotor d'entraînement annulaire ou en forme de disque est intégré dans une roue à ailettes, de préférence en matière synthétique.

19. Machine rotative selon l'une des revendications 1 à 18, réalisée comme pompe rotative, où le signal de la position axiale du rotor ou le signal de réglage d'une installation de réglage, qui est nécessaire pour la stabilisation axiale du rotor, est utilisé pour déterminer la pression de la pompe.

20. Machine rotative selon l'une des revendications 1 à 18, réalisée comme pompe rotative, avec des moyens pour déterminer l'écoulement du fluide à travers la pompe à partir du signal de la position axiale du rotor, de la vitesse de rotation du rotor ainsi que du courant d'entraînement.

21. Machine rotative selon l'une des revendications 1 à 20, caractérisée en ce que des moyens sont présents dans le stator qui produisent dans le moteur électrique un flux unipolaire (52, 53, 54, 55) et en ce que le stator comprend un enroulement de champ magnétique tournant à deux pôles par lequel peuvent être commandées des forces magnétiques radiales agissant sur le rotor, et en ce que le stator comprend un enroulement de champ magnétique tournant supplémentaire avec un nombre de pôles de paires p>=2 par lequel peut être produit un champ tournant pour entraîner le rotor.

22. Machine rotative selon la revendication 21, caractérisée en ce que le flux unipolaire dans le moteur électrique est produit par des aimants permanents (50, 51).
